# EUROPEAN PATENT SPECIFICATION

(11) **EP 3 735 315 B1**
(45) Date of publication and mention of the grant of the patent: **04.03.2026**
(21) Application number: 19735767.6
(22) Date of filing: 07.01.2019
(51) Int. Cl.: G01N 30/88, G01N 30/06, B01D 71/02, G01N 33/48, G01N 33/53, B01D 67/00, B01D 69/00

(54) **SAMPLE PREPARATION AND FLOW-THROUGH SENSORS USING FUNCTIONALIZED SILICON NANOMEMBRANES**
PROBENVORBEREITUNG UND DURCHFLUSSSENSOREN UNTER VERWENDUNG VON FUNKTIONALISIERTEN SILIZIUMMEMBRANEN
PRÉPARATION D'ÉCHANTILLONS ET CAPTEURS DE CIRCULATION UTILISANT DES NANOMEMBRANES DE SILICIUM FONCTIONNALISÉES

(30) Priority: 05.01.2018 US 201862614221 P
(43) Date of publication of application: 11.11.2020
(73) Proprietor: SiMPore Inc., West Henrietta, NY 14586 (US); University of Rochester, Rochester, NY 14642 (US)
(72) Inventor: CARTER, Jared, A., Rochester, NY 14623 (US); ROUSSIE, James, A., Rochester, NY 14617 (US); MADEJSKI, Gregory, Albion, NY 14411 (US); McGRATH, James, L., Fairport, NY 14450 (US)
(74) Representative: FRKelly
(86) International application number: PCT/US2019/012581
(87) International publication number: WO 2019/136402

(56) References cited:
- EP-A1- 3 146 321
- EP-B1- 3 146 321
- WO-A1-2016/106689
- WO-A1-2016/161400
- US-A1- 2004 228 568
- US-A1- 2006 278 580
- US-A1- 2011 223 424
- US-A1- 2012 178 091
- US-A1- 2013 040 827
- US-A1- 2014 248 642
- US-A1- 2016 003 823
- CARLEN E T ET AL: "Recent progress in microfluidic devices for nucleic acid and antibody assays", PROCEEDINGS OF THE IEEE, IEEE. NEW YORK, US, vol. 91, no. 6, 1 June 2003 (2003-06-01), pages 954 - 975, XP011097418, ISSN: 0018-9219, DOI: 10.1109/JPROC.2003.813569
- OUKHALED A. ET AL.: "Sensing Proteins through Nanopores: Fundamental to Applications", 1 ACS CHEMICAL BIOLOGY, vol. 7, no. 12, 2012, pages 1935 - 1949, XP055362448, DOI: doi:10.1021/cb300449t
- LARSON B. J. ET AL.: "Cold-plasma modification of oxide surfaces for covalent biomolecule attachment", BIOSENSORS AND BIOELECTRONICS, vol. 21, no. 5, 2005, pages 796 - 801, XP005122226, DOI: doi:10.1016/j.bios.2005.02.005
- RONG G. ET AL.: "Nanoscale porous silicon waveguide for label-free DNA sensing", BIOSENSORS & BIOELECTRONICS, vol. 23, no. issue 10, 2008, pages 1572 - 1576, XP022593630, DOI: doi:10.1016/j.bios.2008.01.017

## Description

### FIELD OF THE DISCLOSURE

The present disclosure relates to uses of silicon membranes in methods of preparing, detecting, or assaying an analyte of a sample.

### BACKGROUND OF THE DISCLOSURE

For many analytical techniques, such as assays that identify, detect, and/or quantify analytes of interest, there is a reliance on selective capture of the analyte by an affinity agent. In general, the affinity agents are bound to a surface to which the sample bearing the analytes is presented, such that the affinity agents can selectively bind the analytes and thus capture them out of the sample. These steps are often performed for purposes of performing a diagnostic assay or test.

Due to thermodynamic and chemical factors (e.g., van der Waals interactions, entropy, etc.), there is an inherent steric limitation to the amount of analyte that can be captured by surface-bound affinity agents. Further, there are kinetic factors that limit such capture, which may be described as diffusion rate-limited capture, for the reasons previously listed.

The analyte binding kinetics within a flow-over fluidic device (i.e., a non-porous device) are diffusion-limited. A flow-through fluidic device may improve the capture of analytes. However, methods to date for flow-through capture suffer from low throughput and are uncoupled from the analytical means for identifying, detecting, and/or quantifying the analyte once captured.

Existing polymeric membranes (e.g., well-known polycarbonate, cellulose, or polyethersulfone) possess insufficient optical transparency and are not sufficiently permeable for flow-through sensor assays. Other non-polymeric membranes used in flow-through fluidic devices suffer from a number of limitations; e.g., porous silicon or anodized alumina. Due to the tens of micron thickness of such membranes, elaborate optical modalities and associated instrumentation complexity are required for detection and quantifying analytes within these media (e.g., optical cavity resonance and confocal microscopy, respectively). Moreover, neither of these optical modalities and their related instrument complexity is compatible with point-of-care or lab-on-a-chip formats that are desirable for current diagnostic applications.

Thus, there is an unmet need for a thin, permeable, and optically transparent membrane that can be modified with affinity agents, and thus permit efficient analyte capture and highly sensitive analyte detection with low complexity instrumentation.

WO2016106689A1 discloses methods and systems for assaying the presence of a target nucleic acid molecule in a sample having or suspected of having the target nucleic acid molecule.

US2016003823A1 discloses systems and methods for sample processing.

US2012178091A1 discloses assay cartridges that have purification, reaction, and detection zones and other fluidic components which can include sample chambers, waste chambers, conduits, vents, reagent chambers, reconstitution chambers and the like. The assay cartridges are used to conduct multiplexed nucleic acid measurements.

US2004228568A1 discloses a porous silicon filter capable of binding and detecting biological and chemical target molecules in liquid or gas samples. A photonic waveguiding silicon filter with chemical and/or biological anchors covalently attached to the pore walls binds target molecules. The system uses transmission curve engineering principles to allow measurements to be made in situ and in real time to detect the presence of various target molecules and calculate the concentration of bound target.

US2006278580A1 discloses a process for forming a porous nanoscale membrane.

Oukhaled et al ACS Chem. Biol. 2012, 7, 12, 1935-1949 relates to Sensing Proteins through Nanopores: Fundamental to Applications.

US2013040827A1 discloses methods of nucleic acid sequencing that use nanopores to detect and/or measure amounts of compounds, such as products or byproducts of nucleic acid sequencing reactions, and to the determination of a nucleotide sequence using such detection and/or measurement.

US2014248642A1 discloses apparatus for the quantitative analysis of an analyte in a sample.

Larson et al, Biosensors and Bioelectronics, Volume 21, Issue 5, 2005, Pages 796-801, discloses Cold-plasma modification of oxide surfaces for covalent biomolecule attachment.

### SUMMARY OF THE PRESENT DISCLOSURE

The present invention is defined in the appended claims. Provided is a method of preparing, detecting, or assaying an analyte of a sample, comprising:
contacting the sample with a fluidic device comprising a functionalized silicon membrane, wherein the fluidic device isolates one or more analytes of interest from the sample; wherein the functionalized silicon membrane is a functionalized silicon nanomembrane chosen from a nanoporous silicon nitride membrane, a microporous silicon nitride membrane, a microslit silicon nitride membrane, and a microporous silicon oxide membrane;
passing a wash solution through the fluidic device; and
   i) eluting the isolated analyte of interest;
      transferring the eluted analyte of interest to a storage vessel or analytical instrument; and
      performing one or more analytical assays on the eluted analyte of interest; or
   ii) passing a solution of one or more detection reagents through the fluidic device; and
      measuring a signal of one or more detection reagents; or
   iii) extracting nucleic acids from the analyte captured by the fluidic device;
      performing a sequencing and/or amplification reaction, wherein reagents for such reactions are passed into the fluidic device;
      passing a solution of one or more detection reagents through the device;
      measuring a signal of one or more amplification and/or sequencing reaction products; and
the method further comprising a step of functionalizing a silicon membrane to form the functionalized silicon membrane by:
contacting the silicon membrane with a chemical oxidation reagent;
contacting the silicon membrane with an epihalohydrin;
contacting the silicon membrane with a catalyst; and
contacting the silicon membrane with one or more biomolecules.

The present disclosure describes fluidic devices for sample preparation and biosensors, where the fluidic devices incorporate functionalized silicon membranes. For purposes of this disclosure, a silicon membrane may be referred to as a nanomembrane and may comprise a plurality of nanopores, micropores, or microslits, wherein the plurality of nanopores, micropores, or microslits fluidically connected one or more membrane surface to an opposing one or more second membrane surface and at least one aperture. For example, such functionalized silicon membranes (e.g., nanomembranes) are nanometer-thick, endowing them with high permeability and optical transparency. Such functionalized silicon membranes (e.g., nanomembranes) can overcome one or more of the limitations associated with other types of polymeric and non-polymeric membranes for flow-through fluidic device applications. The high permeability of functionalized silicon membranes (e.g., nanomembranes) endows them with beneficial convective flow capture of analytes, while their optical transparency endows them with compatibility with a wide range of optical modalities for sensitive detection and/or quantification of captured analytes.

The present disclosure describes flow-through analyte capture and release (i.e., sample preparation) fluidic devices and flow-through analyte capture and detection (i.e., diagnostic assay) combination fluidic devices. The present disclosure further describes functionalized silicon membranes (e.g., nanomembranes) incorporated into such fluidic devices. The present disclosure further describes methods and kits for use of such fluidic devices.

The present disclosure provides methods, uses, and kits. The methods, uses, and kits use functionalized silicon membranes (e.g., nanomembranes) for filtration-related applications, such as sample preparation and diagnostic assays, within fluidic devices.

### BRIEF DESCRIPTION OF THE FIGURES

For a fuller understanding of the nature and objects of the disclosure, reference should be made to the following detailed description taken in conjunction with the accompanying figures.

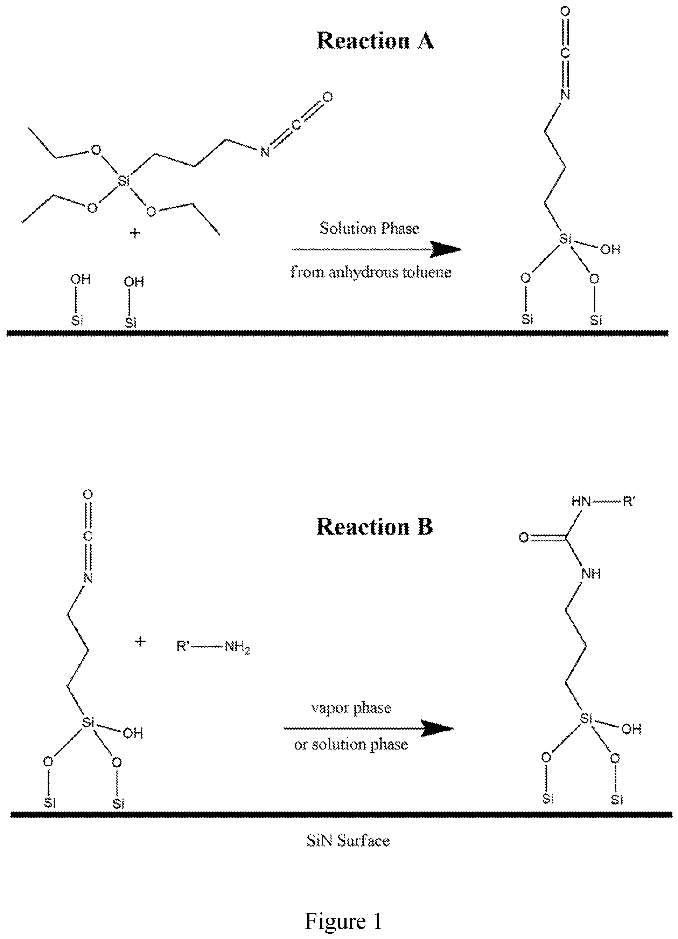

Figure 1 shows a two-step reaction mechanism which demonstrates covalent modification via a classical silane condensation reaction onto silicon-rich SiN via selective modification of silicon oxide terminal groups. "Reaction A" characterizes the bulk deposition of an amine-reactive (isocyanate functional group) trialkoxy silane onto previously oxidized silicon nitride. In this mechanism the terminal silicon atoms are oxidized, and provide a surface reactive to the silane via dehydration of the alkoxy leaving group (in this instance ethanol). "Reaction B" demonstrates the subsequent modification of the surface by an any primary amine containing species yield a stable urea linker mechanism under a variety of reaction conditions (though favored under slightly basic conditions)

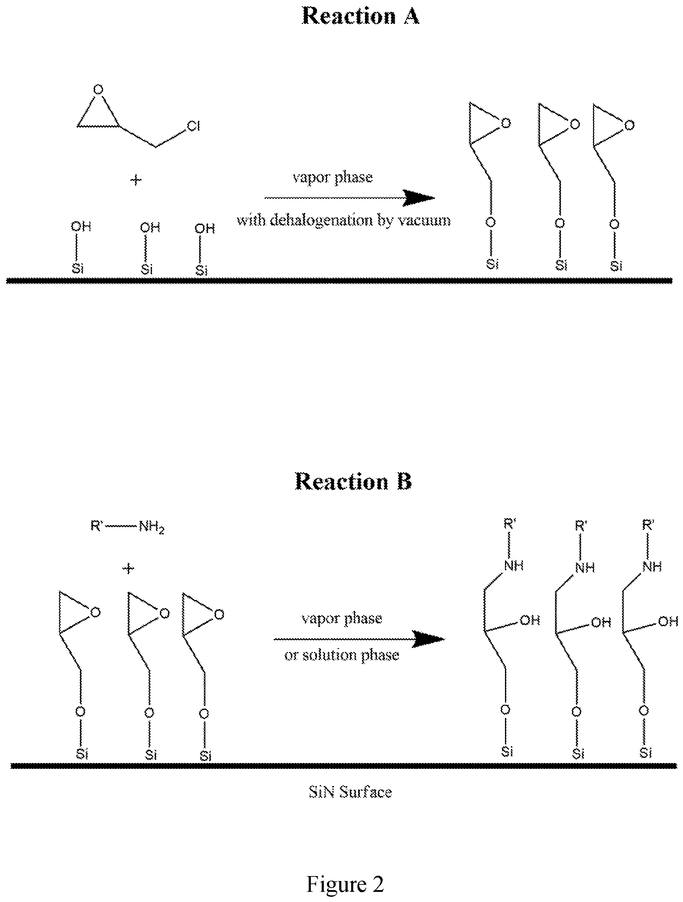

Figure 2 shows a gaseous phase derivatization of previously oxidized Si-rich SiN surfaces using epihalohydrin as a surface linker yielding a terminal epoxide group. "Reaction A" demonstrates the covalent decoration of SiOH group on the SiN surface by epichlorohydrin which reacts via a ring-opening reaction of the epoxide, followed by the reformation of the epoxide ring by subsequent dehalogenation under vacuum. "Reaction B" demonstrates the subsequent modification of the surface by an any primary amine containing species yield a stable urea linker mechanism under a variety of reaction conditions

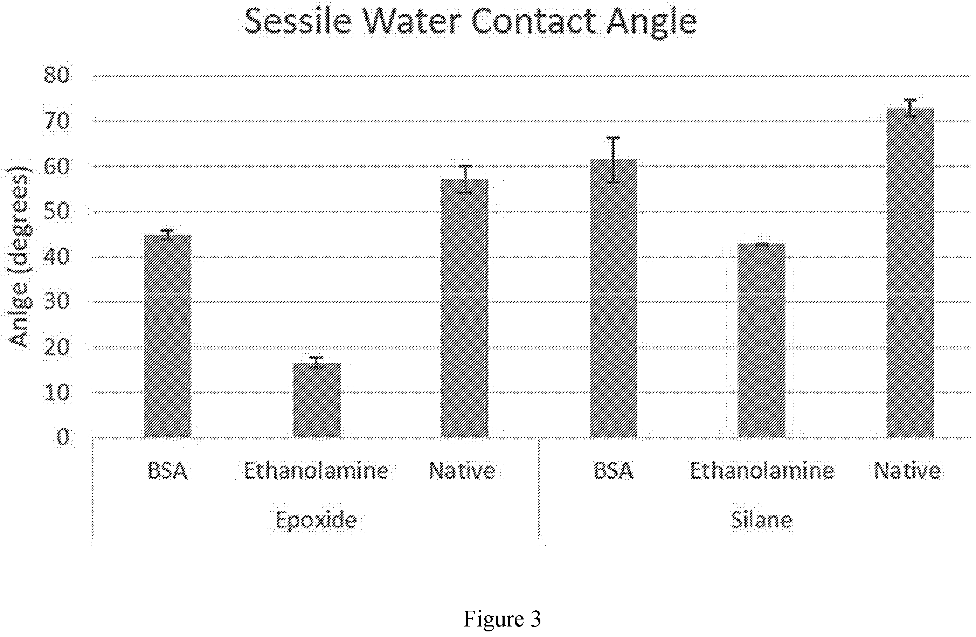

Figure 3 shows sessile water contact angle data for films prepared using the reaction mechanisms detailed in Figure 1 (silane-based chemistry) and Figure 2 (epoxidation-based chemistry). Films of both varieties were either further reacted with a purified protein (bovine serum albumin), a non-fouling group (ethanolamine), or unchanged (native). In the native condition, water contact angles collected demonstrate a significant increase in surface hydrophobicity consistent with the decoration of carbon-rich surface groups. Wetting angles decrease considerably with subsequent treatment via both a protein and ethanolamine, consistent with the increase in hydrophilic species on the underlying films.

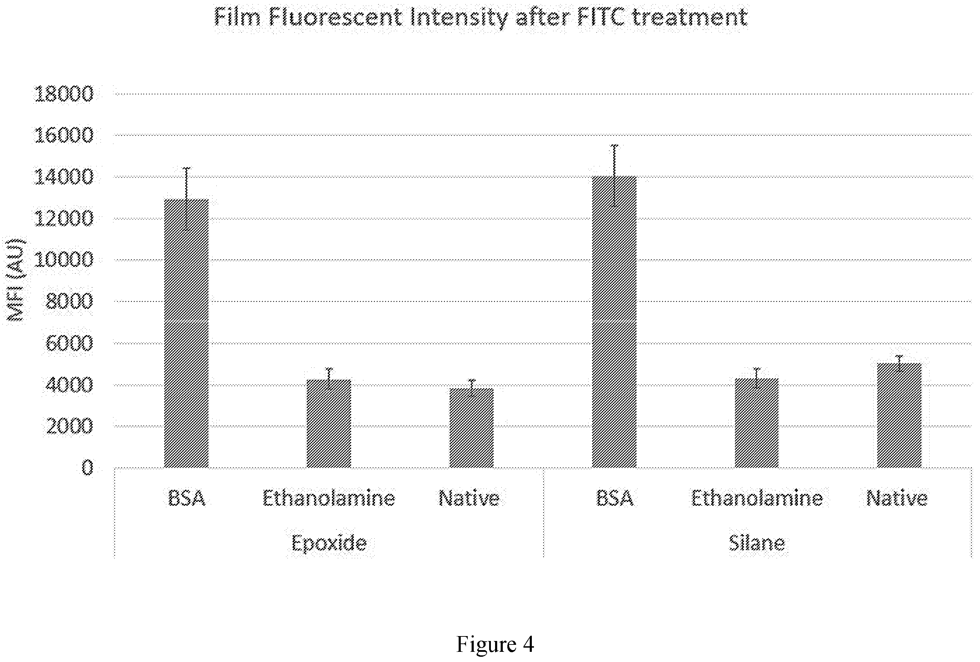

Figure 4 shows fluorescent labeling of the various surfaces further derivatized in Figure 3 via fluorescein isocyanate under basic aqueous conditions. Fluorescent labeling of each surface type confirms the presence of the primary amine-rich purified protein (BSA) and no labeling of the native or ethanolamine-treated surface (consistent with the predicted surface composition of all films).

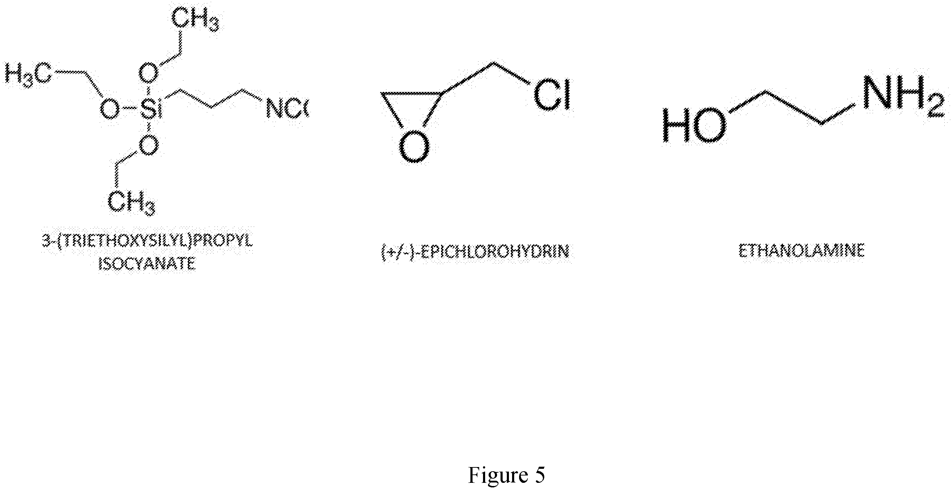

Figure 5 shows structures of the surface derivatizing chemistries used in Example 1, including an isocyante-functional silane (3-(triethoxysilyl)-propyl isocyanate), epoxidation reagent (epichlorohydrin), and a terminal non-fouling group (ethanolamine).

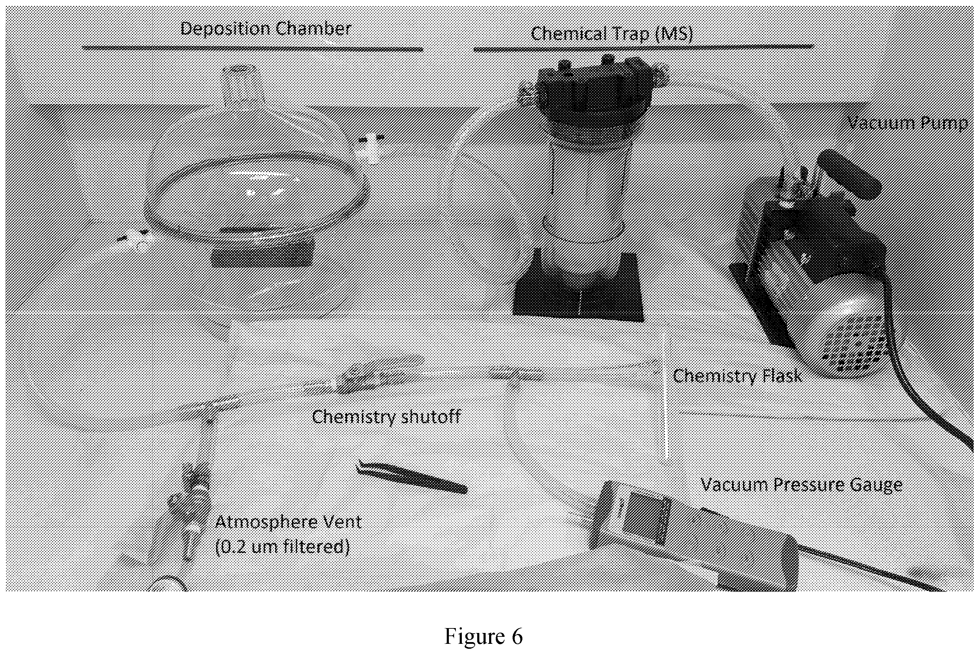

Figure 6 shows a basic system for the gaseous-phase covalent modification of previously-oxidized silicon nitride membranes. The system is generally composite of a vacuum pump, a chemical trap (filled with molecular sieves to getter waste reaction products and unreacted chemistry), a deposition chamber, a system vent to atmosphere, a chemistry flask, and a pressure monitor. A series of valves allows the isolation of each system element to control the flow of gases through the deposition chamber.

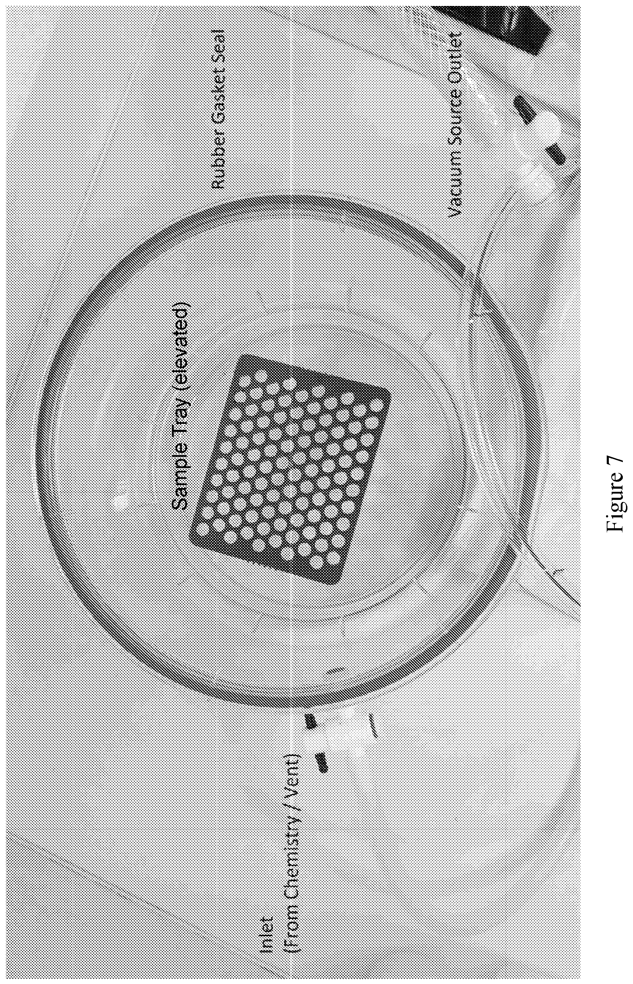

Figure 7 shows a detail of the deposition system shown in Figure 6, which shows the perforated polypropylene sample tray, elevated to promote gaseous chemistry flow across and through the SiN membranes. The chamber dome itself is sealed with a perimeter gasket and may be accessed by two valve ports for vacuum and chemistry access to the chamber.

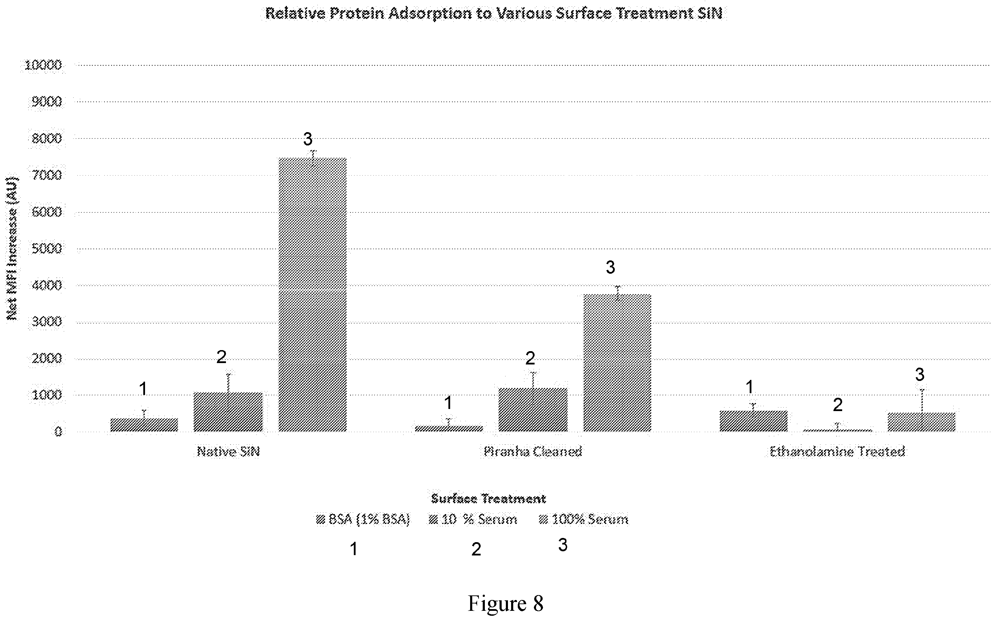

Figure 8 shows relative protein adsorption to various Silicon Nitride films in either a native state, Pre-cleaned with piranha, or ethanolamine coated using the reaction chemistry described in Figure 2. All films evaluated were exposed to solutions of dilute (10% in PBS), neat adult bovine serum, or 1% serum albumin in PBS for 24 hours at room temperature. Nonspecifically adsorbed protein films were fluorescently labeled using FITC under slightly basic aqueous conditions, then background corrected against non-protein exposed control SiN membranes. These data demonstrate surface functionalization and termination with ethanolamine increases repulsion of protein species likely by maintaining a neutral surface charge and tightly bonded water layer at the surface interface.

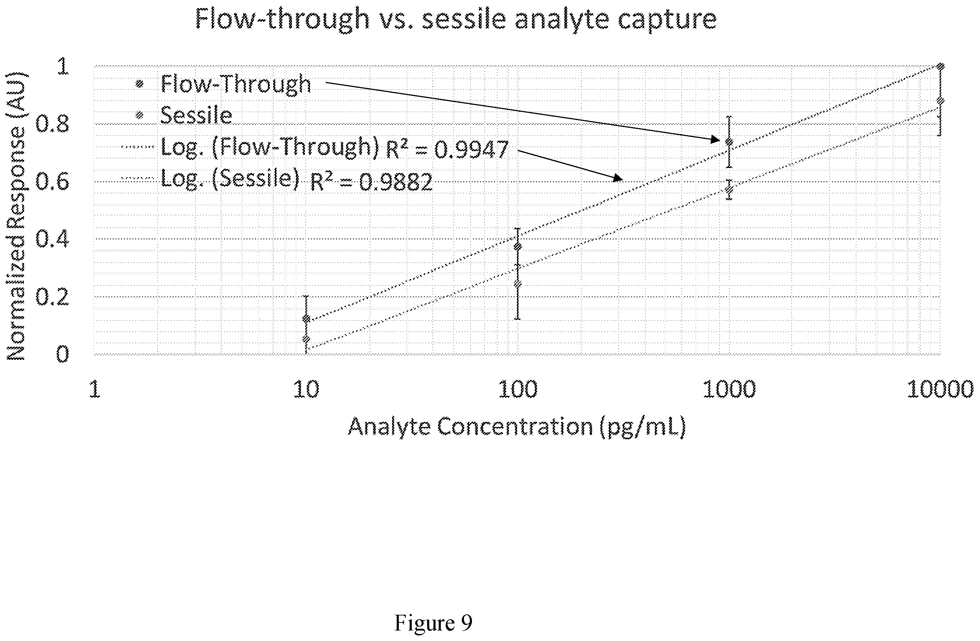

Figure 9 shows detection data demonstrating a net signal increase via the flow-through sensor format as opposed to a standard sessile format assay. In this experiment, Streptavidin-Alkaline Phosphatase was used as the analyte captured via membranes functionalized with PEG-Biotin using either stationary target incubation (orange data) or when the target solution is actively passed through the membrane via centrifugation. For all data, n = 2 replicate sensors were used and n= 3 subsections of the membrane surface area were analyzed.

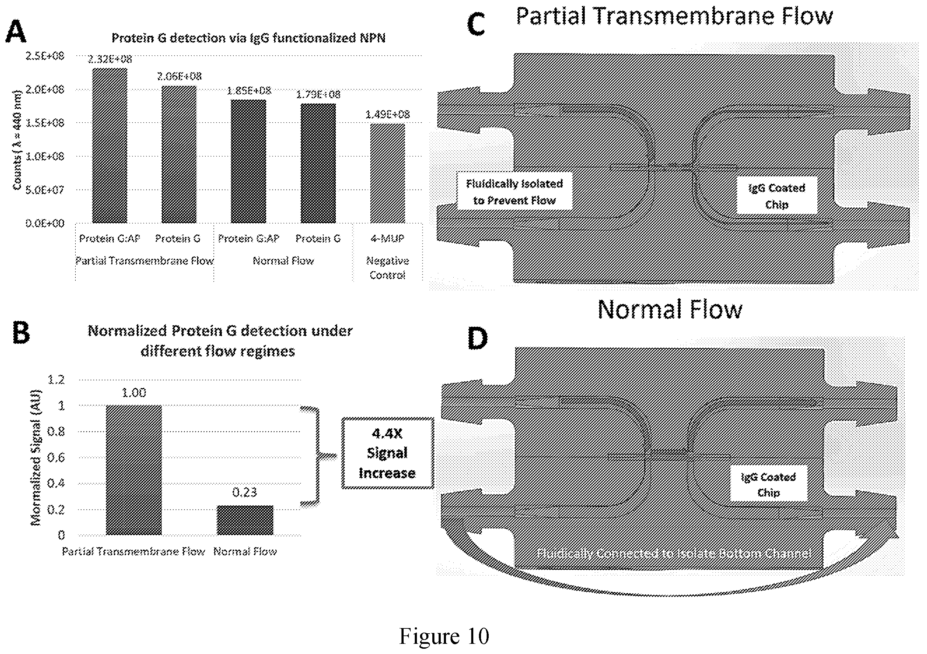

Figure 10 depicts specific capture and detection of a representative protein using a probe-functionalized nanoporous membrane surface. In this experiment, an epichlorohydrin reaction was used to attach immunoglobulin G (IgG) to the membrane, which was then used to capture a recombinant IgG-binding specific protein (Protein G, native or Alkaline Phosphatase conjugated). (A) Detection results for the various IgG coated membrane exposure conditions with error bars corresponding to the standard error of the mean response measured from two replicate sensors.. (B) Normalized Protein G detection under partial transmembrane and normal flow, showing an average 4.8-fold increase in detection signal for n=2 replicate experiments using partial transmembrane flow through the sensor. Flow diagram schematics for (C) the partial transmembrane flow sensor and (D) the normal flow sensor used in this experiment.

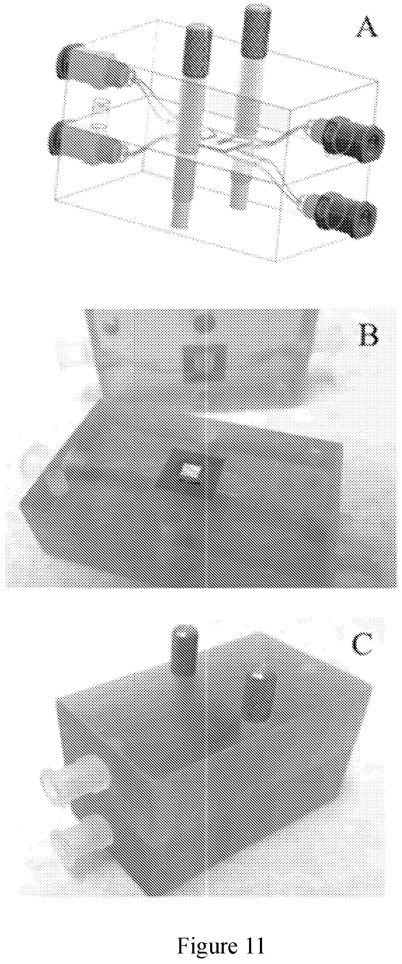

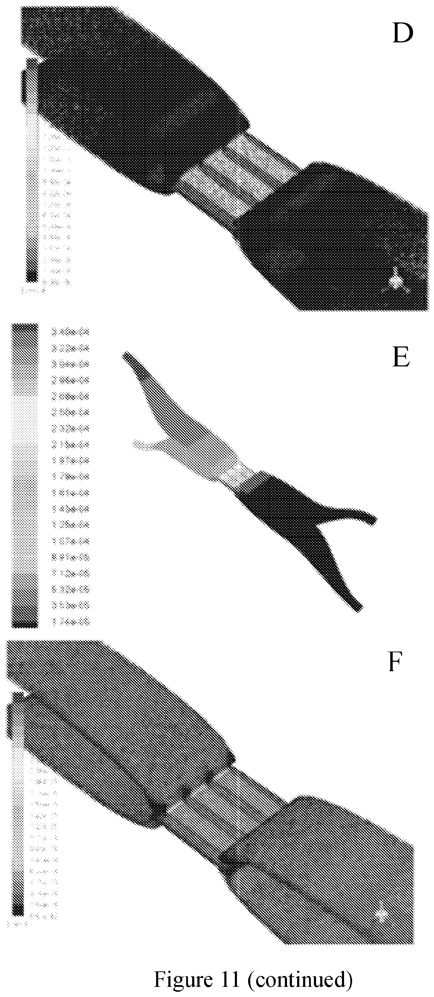

Figure 11 shows a tangential flow-based fluidic device for incorporating nanomembrane filters. A prototype Fluidic Module with polycarbonate fluidic channels in the body and elastomeric gaskets for filter integration was fabricated by 3D-printing. CAD modeling software was used to render a prototype device (A) suitable for multi-material 3D-printing (B-C). Computational fluid dynamics analysis was performed on the design to verify surface velocities (D), system pressure (E) and sheer stress (F) to ensure such exemplary prototypes would be suitable fluidic devices for the methods of the present disclosure.

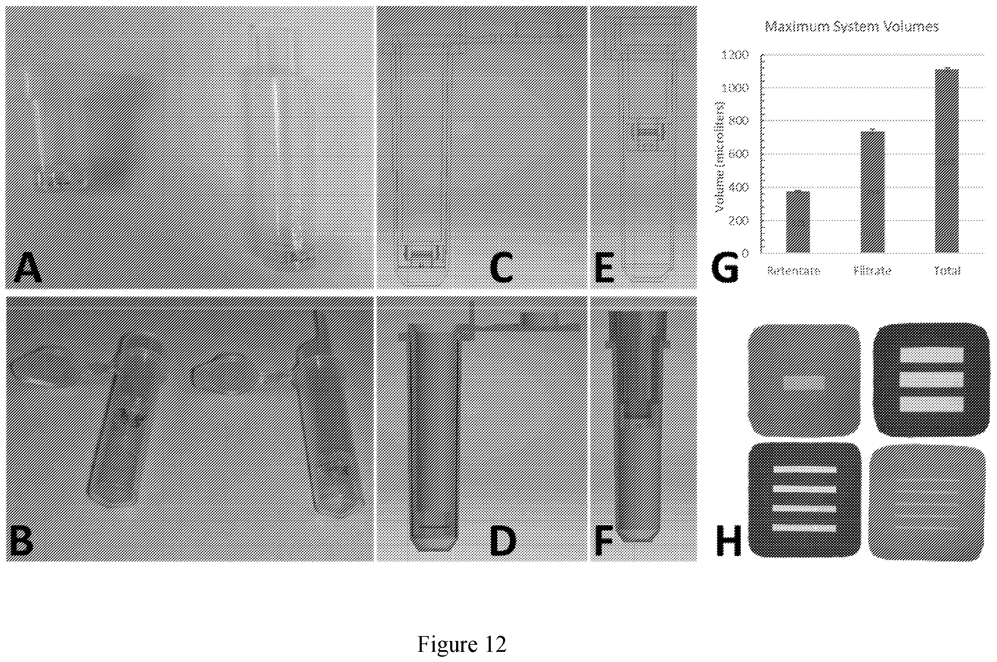

Figure 12 shows a representative fluidic device incorporating a nanomembrane filter, wherein the nanomembrane filter is integrated into a centrifuge tube insert fluidic device for dead-end (normal) flow filtration purposes. (A, B, C, D, E, and F) shows representative filter devices incorporating silicon nitride membranes that may employ one or more non-fouling coatings as previously described. (H) shows a series of representative nanomembranes fabricated using similar fabrication processes.

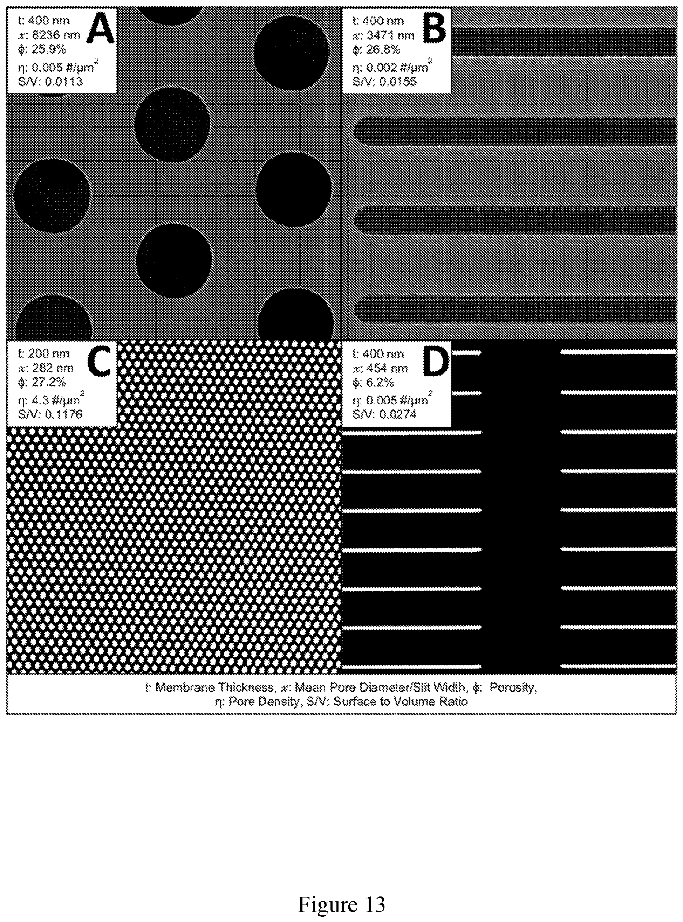

Figure 13 shows images taken via Electron Microscopy of a range of Silicon Nitride membranes. (A) shows a 400 nm thick microporous SiN membrane of 25.9% porosity decorated with 8.2-micron diameter pores at regular intervals. (B) shows a 400 nm thick microslit membrane of 26.8% porosity with 3.5-micron wide slits. (C) shows a 200 nm thick SiN membrane of 27.2% porosity and 282 nm pores at regular intervals. Finally, (D) shows a 400 nm SiN membrane of 6.2% porosity comprised of 454 nm wide slits.

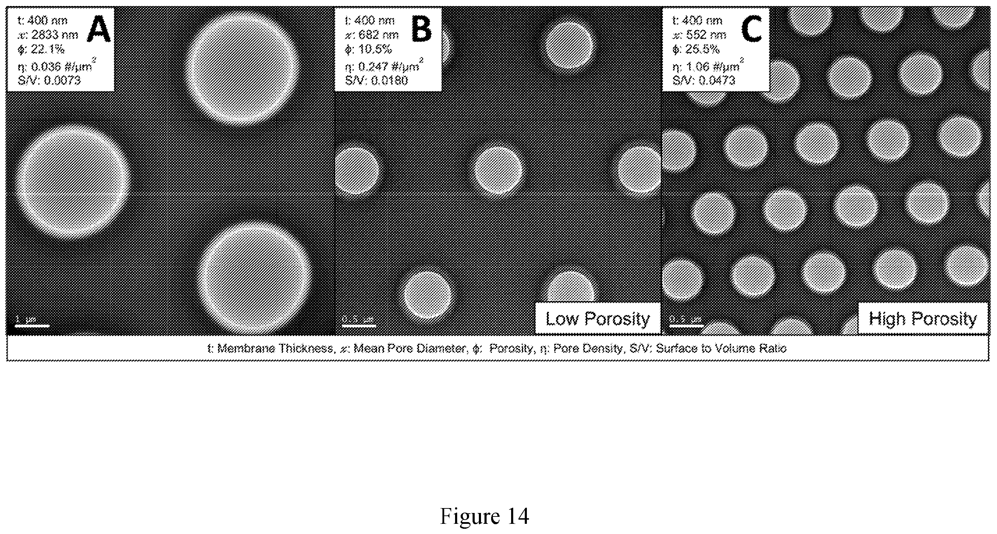

Figure 14 shows a further image study of micropores as evaluated by electron microscopy. (A) shows a 400 nm thick SiN membrane of 22.1% porosity containing 2.8-micron diameter pores. (B) shows a 400 nm thick SiN membrane of 10.5% porosity containing 682 nm diameter pores. (C) shows a 400 nm thick SiN membrane of 25.5% porosity containing 552 nm diameter pores.

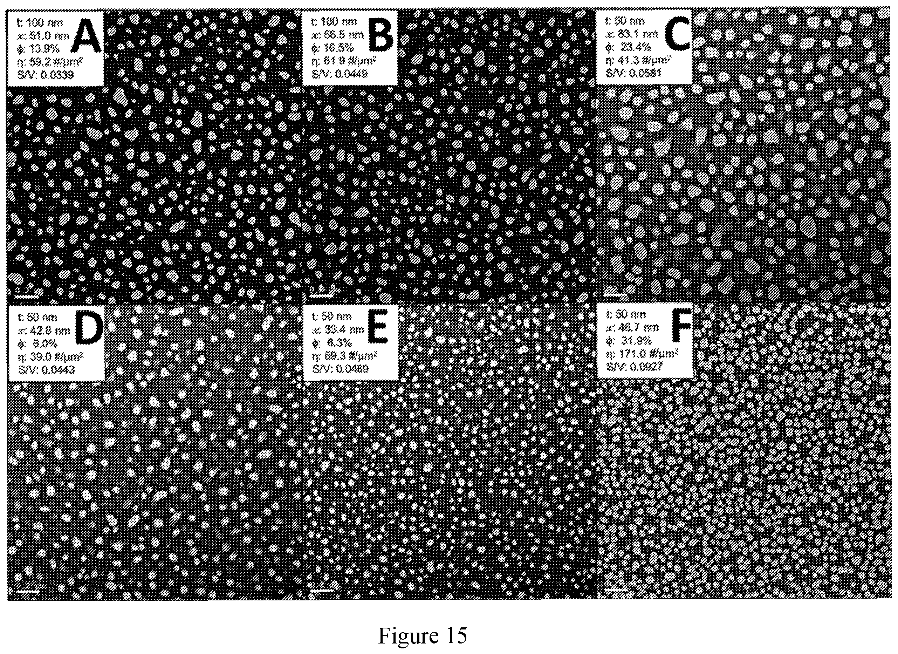

Figure 15 shows a series of nanoporous nitride membranes fabricated using a range of membrane thicknesses, pore diameters, and porosities. (A, B) show a series of 100 nm thick membranes decorated with either 51 nm pores and 13.9% porosity, or 56.5 nm pores and 16.5% porosity respectively. Images (C, D, E, and F) show a series of nanomembranes of 50 nm nominal thickness decorated with a range of pore diameters and porosities as follows [C; 83 nm pores, 23.4% porosity. D; 42.8 nm pores, 6% porosity. E; 33.4 nm pores, 6.3% porosity. F; 46.7 nm pores, 31.9% porosity].

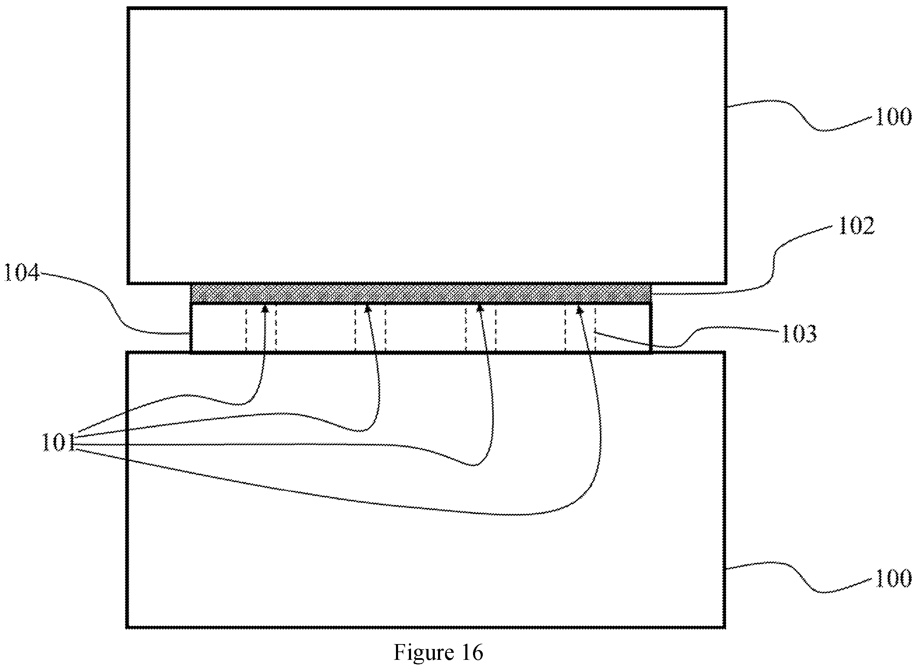

Figure 16 shows a schematic representation of a fluidic device comprising a silicon membrane (e.g., nanomembrane) of the present disclosure. The figures shows fluidic channels/chambers (100); membrane surfaces (101); a porous membrane (102); apertures (103); and a substrate (104).

### DETAILED DESCRIPTION OF THE DISCLOSURE

Although the disclosed subject matter will be described in terms of certain examples, other examples, including examples that do not provide all of the benefits and features set forth herein, are also within the scope of this disclosure. Various structural, logical, and process step changes may be made without departing from the scope of the disclosure. The invention is defined by the appended claims.

Ranges of values are disclosed herein. The ranges set out an example of a lower limit value and an example of an upper limit value. Unless otherwise stated, the ranges include all values to the magnitude of the smallest value (either lower limit value or upper limit value) and ranges between the values of the stated range.

As used herein, unless otherwise indicated, the term "aliphatic" refers to branched or unbranched hydrocarbon groups that, optionally, contain one or more degrees of unsaturation. Degrees of unsaturation include, but are not limited to, alkenyl groups/moieties, alkynyl groups/moieties, and cyclic aliphatic groups/moieties. For example, the aliphatic group can be a C₁ to C₁₈ aliphatic group, including all integer numbers of carbons and ranges of numbers of carbons therebetween. The aliphatic group can be unsubstituted or substituted with one or more substituent. Examples of substituents include, but are not limited to, various substituents such as, for example, halogens (-F, -Cl, -Br, and -I), additional aliphatic groups (e.g., alkenes, alkynes), aryl groups, alkoxides, carboxylates, carboxylic acids, ether groups, silane groups, amine groups, thiol/sulfhydryl groups, isothiocyanate groups, epoxide groups, maleimide groups, succinimidyl groups, anhydride groups, mercaptan groups, hydrazine groups, N-glycan groups, O-glycan groups, and the like, and combinations thereof.

As used herein, unless otherwise indicated, the term "alkyl" refers to branched or unbranched saturated hydrocarbon groups. Examples of alkyl groups include, but are not limited to, methyl groups, ethyl groups, propyl groups, butyl groups, isopropyl groups, tert-butyl groups, and the like. For example, the alkyl group can be a C₁ to C₁₈ alkyl group, including all integer numbers of carbons and ranges of numbers of carbons therebetween,. The alkyl group can be unsubstituted or substituted with one or more substituent. Examples of substituents include, but are not limited to, various substituents such as, for example, halogens (-F, -Cl, -Br, and -I), aliphatic groups (e.g., alkyl groups, alkenyl groups, alkynyl groups), aryl groups, alkoxide groups, carboxylate groups, carboxylic acids, ether groups, silane groups, amine groups, thiol/sulfhydryl groups, isothiocyanate groups, epoxide groups, maleimide groups, succinimidyl groups, anhydride groups, mercaptan groups, hydrazine groups, N-glycan groups, O-glycan groups, and the like, and combinations thereof.

The present disclosure provides methods, uses, and kits. The methods, uses, and kits use functionalized silicon membranes (e.g., nanomembranes) for filtration-related applications, such as sample preparation and diagnostic assays, within fluidic devices.

The present disclosure describes flow-through analyte capture and release (i.e., sample preparation) fluidic devices and flow-through analyte capture and detection (i.e., diagnostic assay) combination fluidic devices. The present disclosure further describes functionalized silicon membranes (e.g., nanomembranes) incorporated into such fluidic devices. For purposes of this disclosure, a silicon membrane may be referred to as a nanomembrane and may comprise a plurality of nanopores, micropores, or microslits, wherein the plurality of nanopores, micropores, or microslits are fluidically connect one or more membrane surface to an opposing one or more second membrane surface and at least one aperture. The present disclosure further describes methods and kits for use of such fluidic devices.

Description of silicon membranes (e.g., nanomembranes) may also refer to description of functionalized silicon membranes (e.g., nanomembranes) and the term silicon membrane may be used when referring to functionalized silicon membrane (e.g., nanomembrane), including singular and plural forms.

The present disclosure describes fluidic devices for sample preparation and biosensors, where the fluidic devices incorporate functionalized silicon membranes (e.g., nanomembranes). For example, such functionalized silicon membranes are nanometer-thick, endowing them with high permeability and optical transparency. Such functionalized silicon membranes can overcome one or more of the limitations associated with other types of polymeric and non-polymeric membranes for flow-through fluidic device applications. The high permeability of functionalized silicon membranes endows them with beneficial convective flow capture of analytes, while their optical transparency endows them with compatibility with a wide range of optical modalities for sensitive detection and/or quantification of capture analytes.

The present methods use flow-through capture surfaces which are not diffusion rate limited. Without intending to be bound by any particular theory, flow-through capture surfaces can offer improved means for selective capture of analytes from samples. It is considered that they derive benefits of convective flow of analyte over the surface-bound affinity agents.

The present disclosure provides porous devices functionalized with affinity agents that are expected to provide more favorable analyte binding kinetics due to convection of sample fluids (bearing the analyte of interest) that flow-through the sample binding aspects. The advantageous surface area-to-volume ratio offered by incorporation of porous membranes into fluidic devices for sample preparation and/or diagnostic assays are expected to enable performance benefits for flow-through sensor applications. The thin porous membranes of the present disclosure, which offer desirable permeability and optical transmission, can be readily functionalized with affinity agents, and offer a means for coupling efficient analyte capture and analyte detection within one medium.

In an aspect, the present disclosure provides methods. The methods can be carried out using devices comprising one or more functionalized silicon membranes (e.g., nanomembranes) described herein. For example, the methods are sample preparation methods or analytical assays (e.g., a portion of or a complete analytical assay).

In an example, sample preparation comprises contacting a sample solution with the silicon membrane functionalized with one or more coating, wherein at least one of the coatings comprises a biomolecule (e.g., affinity moiety, molecular recognition agent, and/or the like) for capturing a species of interest, which is attached to the membrane via one or more covalent bonds. Such a filtration device would be intended as a means for selective isolation of one or more species of interest for the purposes of performing a downstream or subsequent post-isolation analytical assay (i.e., sample preparation upstream of such assays). Following removal of the sample solution, the captured species may be eluted or released from the membrane. The fluidic devices for sample preparation may be tangential or normal flow devices as described herein. Biomolecules and other terminal groups are not passively coated (e.g., physisorbed and/or chemisorbed) on the silicon membrane (e.g., nanomembrane).

For purposes of this disclosure, the functionalization of membranes (e.g., nanomembranes) with aliphatic (e.g., alkyl) containing terminal groups should be considered indirect covalent bonding via any of the functionalization reactions described herein. The modification with aliphatic (e.g., alkyl) containing terminal groups is not direct but rather indirect, wherein any aliphatic or alkyl containing group is reacted with the functionalization groups disclosed herein (e.g., epihalohydrin or bifunctional aldehyde or silane) and not reacted directly with chemically activated membrane surface reactive groups (e.g., -OH, - NH₂, and the like).

In various examples, the elution or release of captured species comprises chemical, mechanical or thermal denaturation, reverse flow of that initially used for capture, or may use a liable bond within the linker moiety, wherein the liable bond is readily broken upon some triggering event (e.g., UV irradiation, chemical reaction, and the like). The eluted or released species could be directed into storage or collection vessel for any number of downstream purposes.

A method may be a method of preparing a sample for an analytical assay. In an example, a method of preparing a sample for an analytical assay comprises: contacting the sample with a fluidic device, wherein the fluidic device isolates one or more analyte of interest from the sample; passing wash solution through the fluidic device; eluting the isolated analyte of interest; transferring the eluted analyte of interest to a storage vessel or analytical instrument; and performing one or more analytical assays on the analyte of interest.

In various examples, the one or more analytical assays is performed on eluted and transferred analytes to identify and quantify the presence or absence of any specific analyte(s) of interest. As examples, these assays include, but are not limited to, a sequencing reaction, an amplification reaction, polymerase chain reaction (PCR), reverse transcriptase-polymerase chain reaction (RT-PCR), ligase chain reaction (LCR), loop-mediated isothermal amplification (LAMP), Taqman^{™} PCR, Northern blotting, Southern blotting, fluorescent hybridization, enzymatic treatment, labeling with secondary biomolecules, enzyme-linked immunosorbent assay, Western blotting, immunoprecipitation, fluorescence-activated sorting, optical imaging, electron microscopy, surface plasmon resonance, Raman spectroscopy, microcalorimetry, interferometry, nanopore-based resistive pulse sensing, or arrayed imaging reflectometry, quartz crystal microbalance, impedance-derived capacitance spectroscopy, electrochemical redox impedance capacitive spectroscopy, and the like, or any combination of the preceding assays. If multiple biomolecules are used to capture two or more analytes, then assays for multiplex detection could be used to distinguish, identify, and quantify multiple analytes or multiple detection reagents used to quantify the multiple analytes using the same assay. Other possible assays known in the art are also suitable.

In an example, the fluidic device comprises a filtration device configured to perform an analytical, diagnostic, and liquid biopsy assay, and is referred to as a flow-through sensor.

In an example, performing a diagnostic assay comprises contacting a sample solution with a functionalized membrane (e.g., nanomembrane) by tangential or normal flow (as described herein). The silicon membrane (e.g., nanomembrane) is functionalized with one or more biomolecules for selective capture of analytes of interest and a number of analytical modalities could be subsequently applied for purposes of carrying out the diagnostic assay. The fluidic device could be configured to carry out all the required steps to achieve the entire diagnostic workflow. The fluidic device may be configured to detect and quantify the presence of one or multiple analytes within a sample, and such detection and/or quantification can comprise using one assay or multiple assays (i.e., multiplex assays).

In an example, a method of detecting an analyte of a sample comprises: contacting the sample with a fluidic device, where the fluidic device isolates the one or more analyte of interest from the sample; passing wash solution through the fluidic device; passing solution of one or more detection reagent through the device; optionally, passing additional wash solution through the device; and measuring a signal of one or more detection reagent.

In the various examples, the diagnostic assay fluidic device is referred to as a flow-through sensor. Flow-through sensors (e.g., porous devices) enable more favorable analyte binding kinetics due to convection of sample fluids (bearing the analyte of interest) that flow-through the sample binding aspects. In contrast, the analyte binding kinetics within a flow-over diagnostic fluidic device (e.g., a non-porous device) are diffusion-limited. The advantageous surface area-to-volume ratio offered by incorporation of silicon membranes (e.g., nanomembranes) into fluidic devices for diagnostic assays may enable performance benefits for flow-through sensor applications.

As one example of a diagnostic assay, the biofluid may be plasma or serum, and the functionalized membrane (e.g., nanomembrane) may be functionalized with one or more antibody for the analytes of isoforms (i.e., isotypes) of the cardiac troponin protein (e.g., cardia troponin i and/or cardiac troponin t), the detection reagent is one or more antibody-conjugate for one or more of the cardiac troponin isoforms, and the diagnostic assay provides clinical information on cardiac status (e.g., occurrence of a myocardial infarction). One or both (as a combination or ratio) of these cardiac troponin tests could be used for diagnostic or prognostic clinical tests. As another example, the biofluid may be plasma or serum, and the functionalized membrane (e.g., nanomembrane) may be functionalized with one or more antibody for the analytes of the glial S100 calcium-binding protein B (S100B) and/or brain-derived neurotropic factor BDNF), the detection reagent is one or more antibody-conjugate for one or both of these proteins, and the diagnostic assay provides clinical information on acute and/or chronic traumatic encephalogy. One or both (as a combination or ratio) of S110B and/or BDNF could be used for diagnostic or prognostic clinical tests. In these examples, the analytical method could be any of those disclosed herein. Of course, other biofluids, other analytes, and/or detection reagents, and/or analytical methods may be used to diagnose or prognose other specific disease states or health conditions, in either single- or multiplex configurations, and these examples have been provided merely for exemplary purposes.

In an example, a method of performing a diagnostic assay comprises contacting a sample solution with a functionalized membrane (e.g., nanomembrane) by tangential or normal flow, wherein the silicon membrane (e.g., nanomembrane) of the fluidic device is functionalized with at least one non-fouling terminal group as described herein. In various examples, the silicon membranes (e.g., nanomembranes) are not functionalized with biomolecules (e.g., affinity agents, and the like). The filtration properties of the contacting functionalized membrane (e.g., nanomembrane) must be specified such that the analytes of interest are retained, while undesired solutes permeate through the membrane. In general, the analytes of interests are larger than the openings of the membrane (e.g., the diameter of the analytes are larger than the pore diameter of the membrane), while the undesired solutes are smaller than the openings of the membrane (e.g., undesired solute diameter is smaller than the membrane pore diameter). The non-fouling terminal groups of the contacting functionalized membrane (e.g., nanomembrane) promote the removal of such undesired solutes, such as abundant matrix interferents often present in sample solutions, and may also promote membrane wetting during contacting and washing steps of the methods disclosed herein. The addition of detection reagents during subsequent steps of the method and thus provide the means by which the retained analytes are identified by the methods described herein. A non-fouling group is a group that promotes non-fouling of the membrane by maintaining a hydration layer (e.g., hydroxyl groups or zwitterionic groups) or by a hydrophobic surface (e.g., per fluorinated groups), wherein either terminal groups prevent non-specific absorption of sample components. Further, the chemical properties of the hydration layer may reduce surface tension, thus promoting the wetting ability of functionalized membranes (e.g., nanomembranes).

In another example, the sample solution and detection reagent may be added to the fluidic device concurrently, and optionally incubated prior to contact with the non-fouling functionalized silicon membrane (e.g., nanomembrane), such that complexes of analytes of interest and detection reagents are formed, and upon filtration, these complexes are retained by the contacting silicon membrane (e.g., nanomembrane), and undesired solutes permeate through the membrane. In such examples, the filtration properties of contacting silicon membranes (e.g., nanomembranes) should thus be specified to retain the analyte-detection reagent complexes and permeate the undesired solutes. The addition of detection reagents during subsequent steps of the method thus provide the means by which the retained analytes are identified by the methods disclosed herein.

In various examples, the method further comprises using any of the analytical modalities described herein for purposes of carrying out the diagnostic assay. The fluidic device could be configured to carry out all the required steps to achieve the entire alternative diagnostic workflow. The fluidic device may be configured to detect and quantify the presence of one or multiple analytes within a sample, and such detection and/or quantification can comprise using one assay or multiple assays (i.e., multiplex assays). Any optional washing steps as disclosed herein for diagnostics assays may be used in the alternative methods.

In another example, the fluidic device is configured for the purposes of a liquid biopsy assay. Species of genomic diagnostic interest, such as circulating tumor cells, white blood cells, platelets, extracellular/cell-free vesicles (e.g., exosomes, micro vesicles, and the like), nucleosomes, and micro-RNA-protein complexes may be selectively captured from biofluid samples using a fluidic device incorporating an appropriately functionalized silicon membrane (e.g., nanomembrane). Once isolated on the functionalized silicon membrane (e.g., nanomembrane) within the fluidic device, genomic material can be extracted and either transferred to a second capture element of the device for further analysis. Alternatively, the extracted genomic material may be directly interrogated on the membrane. For example, a silicon membrane (e.g., nanomembrane) is functionalized with one or more biomolecule having affinity for one or more species of genomic diagnostic interest (e.g., antibodies or aptamers for circulating tumor cells, white blood cells, platelets, extracellular/cell-free exosomes and/or nucleosomes) and further functionalized with additional biomolecules (e.g., DNA and/or RNA oligonucleotides) that can serve as primers for a subsequent amplification or sequencing reaction (e.g., RT-PCR, PCR, loop-mediated PCR, ligase chain reaction, Taqman^{™} PCR, and the like). The amplification or sequencing reaction products can be detected using detection reagents and optical modalities as disclosed herein.

In an example, a method for performing a liquid biopsy assay comprises: contacting the sample with a fluidic device, where the fluidic device isolates the one or more analyte of interest from the sample; passing wash solution through the fluidic device; extracting nucleic acids from any captured analyte; performing a sequencing and/or amplification reaction, where reagents for such reactions are passed into the fluidic device; optionally, passing additional wash solution through the device; optionally, passing solution of one or more detection reagent through the device; and measuring a signal of one or more amplification and/or sequencing reaction products.

In an example, the second capture element within the fluidic device to which the extracted nucleic acid is transferred, is similarly functionalized with DNA and/or RNA oligonucleotide primers for application or sequencing reactions. This additional element may be a second functionalized silicon membrane (e.g., nanomembrane), a well or reservoir patterned in a polymer or inorganic material, or a polymer or inorganic surface. Additionally, the present disclosure may further comprise methods in which any well, reservoir, polymer, or inorganic second elements may be selectively functionalized in comparison to the functionalized silicon membranes (e.g., nanomembranes) optionally incorporated into such devices.

In another example of a liquid biopsy assay, the analyte species of interest may be surface-expressed proteins (e.g., transmembrane proteins with at least one soluble, surface-exposed portion), such as proteins on the outer surface of circulating normal cells, tumor cells, white blood cells, platelets, extracellular/cell-free vesicles (e.g., exosomes or micro vesicles), or apoptic bodies, among others. In one example, performing a liquid biopsy assay for such surface-expressed proteins follows the methods disclosed herein for performing a diagnostic assays. A silicon membrane (e.g., nanomembrane) is functionalized with one or more biomolecules for selective capture of analytes expressing the surface proteins of interest. The analytical modalities described herein could be subsequently applied for purposes of carrying out the liquid biopsy assay (particularly those modalities appropriate for proteinaceous analytes). The fluidic device could be configured to carry out all the required steps to achieve the entire liquid biopsy or diagnostic workflow. The fluidic device may be configured to detect and quantify the presence of one or multiple analytes within a sample, and such detection and/or quantification can comprise using one assay or multiple assays (i.e., multiplex assays).

For purposes of this disclosure, a liquid biopsy assay is considered to be an analytical method that provides diagnostic or prognostic information regarding a disease or health state, wherein a biofluid sample is used to gather such information. A liquid biopsy may be used in lieu of (i.e., replace) a conventional surgical or procedure tissue biopsy. Such liquid biopsies may also be used to monitor the extent of treatment response to particular therapies used to treat a disease. In an example, a liquid biopsy using blood, plasma, or serum is used in lieu of a surgically obtained tissue biopsy for diagnosing a cancer or assessing response of a cancer to treatment. In another example, a liquid biopsy using urine is used in lieu of a surgically obtained tissue biopsy for diagnosing a renal disease (e.g., chronic kidney disease, glomerulonephritis, and the like) or assessing response of a renal disease to treatment. One or more analyte and/or analytical modality may be used for such liquid biopsies, and in preferred examples, a combination of analytes (i.e., a multiplex assay) offers greater diagnostic, prognostic, and/or treatment response information versus a similar assay with only one of the analytes within a combination set of analytes. For example a multiplex assay comprising a set of two or more analytes may provide greater sensitivity, specificity, and/or greater area under a receiver-operator curve (or the like) than provided by any one analyte alone (the any one analyte being a member of the combination set).

In an example of a liquid biopsy assay, the biofluid sample may be serum, plasma, or urine, and the functionalized membrane (e.g., nanomembrane) may be functionalized with one or more antibody for the analytes of extracellular vesicles or cell-free nucleoprotein particles(e.g., comprising proteins and either DNA or RNA), and the detection reagents and optical modalities of the present disclosure are used, following a sequencing or amplification reaction, to identify and/or quantify the presence of specific nucleic acid sequences within any of these analytes. Such sequences may include, among others, nucleosomal DNA, messenger RNA, micro RNA, and/or long non-coding RNA sequences, any of the foregoing sequences with modifications(e.g., methylated or acetylated nucleotides), or any combinations of any of the preceding analytes and/or modifications. In such examples, the identification and/or quantification of one or more sequences may be clinically useful for diagnosing or prognosing a disease, or for monitoring treatment response. For instance, if the biofluid is either serum or plasma, then these exemplary liquid biopsies may provide clinical information regarding any major organ system such as heart, lung, liver, stomach, kidney, pancreas, nervous, lymphatic, or hematopoietic, among others (e.g., any oncologic, infectious, inflammatory, necrotic, sclerotic, fibrotic (or the like) condition, either acute or chronic, of any of the foregoing systems). As an additional instance, if the biofluid is urine, then these exemplary liquid biopsies may provide clinical information regarding the genitourinary tract (e.g., any oncologic, infectious, inflammatory, necrotic, sclerotic, fibrotic (or the like) condition, either acute or chronic, of the kidney, bladder and/or reproductive systems). Of course, other biofluids and/or other analytes may be used for liquid biopsies to diagnose, prognose, and/or monitor other specific disease states or health conditions, in either single- or multiplex configurations, and these examples have been provided merely for exemplary purposes.

In another example of a liquid biopsy assay, the biofluid sample may be serum, plasma, or urine, and the functionalized membrane (e.g., nanomembrane) may be functionalized with one or more antibody for the analytes of extracellular vesicles, the detection reagent is one or more antibody-conjugate for one or more surface-expressed proteins of such extracellular vesicles, and the optical modalities of the present disclosure are used to identify and/or quantify the presence of specific vesicular surface-expressed proteins. In such examples, the identification and/or quantification of one or more such proteins may be clinically useful for diagnosing or prognosing a disease, or for monitoring treatment response. For instance, if the biofluid is either serum or plasma, then these exemplary liquid biopsies may provide clinical information regarding any major organ system such as heart, lung, liver, stomach, kidney, pancreas, nervous, lymphatic, or hematopoietic, among others (e.g., any oncologic, infectious, inflammatory, necrotic, sclerotic, fibrotic (or the like) condition, either acute or chronic, of any of the foregoing systems). As an additional instance, if the biofluid is urine, then these exemplary liquid biopsies may provide clinical information regarding the genitourinary tract (e.g., any oncologic, infectious, inflammatory, necrotic, sclerotic, fibrotic (or the like) condition, either acute or chronic, of the kidney, bladder and/or reproductive systems). Of course, other biofluids and/or other analytes may be used for liquid biopsies to diagnose, prognose, and/or monitor other specific disease states or health conditions, in either single- or multiplex configurations, and these examples have been provided merely for exemplary purposes.

In various examples, the steps of contacting, washing, eluting, and/or adding detection reagent comprises one of gravity flow, hydrostatic pressure, pumping, vacuum, centrifugation, gas pressurization, normal flow, tangential flow, or combinations thereof. The flow rates, incubation times, and temperatures at which such steps are performed may be specified or controlled as needed for carrying out the method, and may be repeated and/or iterated with any degree of repetition or iteration as desired for carrying out the method. Accordingly, a kit of the present disclosure may comprise fluidic reservoirs, programmable controllers, pumps, actuators, fluidic valves, additional fluidic channels or chambers, and the like, as required for carrying out the methods of the present disclosure.

In various examples, the sample comprises a biological sample, including conditioned cell culture media, cell lysates, venous whole blood, arterial whole blood, plasma, serum, sputum, urine, semen, breath, vaginal fluid, bronchiole fluid, cerebrospinal fluid, bodily secretions, discharges, and/or excretions, as well as swabs and/or aspirates of bodily tissues, and the like. In some examples, an optional pretreatment of the biofluid sample may be carried out prior to carrying out the methods of the present disclosure, such as, for example, low-speed centrifugation of whole blood to remove hemocytes (thus forming a plasma sample), lysis of a population of cells (thus forming a cell lysate), fluidization of a solid sample (thus forming a liquid sample), and other possible pretreatment alternatives. In addition to biological samples, non-biological samples that are compatible with the present disclosure could include samples of water, industrial chemicals, industrial discharges, chemical solutions, pharmaceuticals, food products, milk, air filtrates, volatile organic compounds (e.g., explosives), and the like, and thus include food, environmental and industrial samples.

In various examples, the washing step comprises addition of a buffer solution of specified pH, salt, detergent, carrier biomolecule concentration, and the like, wherein the concentration of buffer components are specified to promote specific interactions or to disrupt non-specific interactions, as required by the methods disclosed herein. For example, the pH may be ≤ 5 or ≥ 9 to disrupt such non-specific interactions. As another example, the salt may be ≥ 500 mM to disrupt such non-specific interactions. As yet another example, a detergent such as Trion X-100, Tween 20, or sodium dodecyl sulfate may be used at a concentration of 0.01% to 0.5% v/v for disrupting such non-specific interactions.

In various examples, the elution step comprises chemical, mechanical or thermal denaturation, or reverse flow of that initially used for contacting the sample, where a fresh bolus of buffer may be flowed to elute the isolated analyte. The elution buffer can comprise addition of a buffer solution of specified pH, salt, detergent, carrier biomolecule concentration, and the like, where the concentration of buffer components are specified to disrupt specific interactions, such that the captured analytes are released. Alternatively, the release of capture analytes may use a liable bond within the affinity moiety, where the liable bond is readily broken upon a treatment (e.g., triggering event, such as, for example, but not limited to, UV irradiation, chemical reaction, and the like). The eluted or released species could be directed into storage or collection vessel for any number of downstream purposes. Accordingly, a kit of the present disclosure may comprise a sonic transducer, a heating element, and/or a light source for the elution, denaturation, and/or photolysis methods of the present disclosure.

In various examples, the selective capture of the analytes of interest comprises a silicon membrane (e.g., nanomembrane) covalently functionalized with one or more biomolecule (e.g., affinity moiety or molecular recognition agent). Non-limiting examples of biomolecules include monoclonal antibodies, polyclonal antibodies, and fragments of monoclonal antibodies, fragments of polyclonal antibodies, DNA aptamers, RNA aptamers, DNA oligonucleotides, RNA oligonucleotides, PNA aptamers, peptides, modified peptide derivatives, lectins, bacteriophages, small molecules, proteins, or combinations thereof.

In various examples, the present disclosure describes multiple methods for measuring a signal of one or more detection reagents captured on flow-through membrane (e.g., nanomembrane) sensors. In some examples, the detection comprises using an optical modality, where the optical signal is derived from the captured detection reagents. In some examples, the detection comprises using a plasmonic-enhanced optical modality, where an enhanced optical signal is derived from the captured detection reagents when used in combination with membranes functionalized with plasmonically active metal conformal coatings (e.g., Au, Pt, Ir, Rh, Ag, and the like). In some examples, the detection comprises using electronic interrogations based on flow-through sensor amperometric or impedimetric methods, where the capture of analytes within functionalized membranes (e.g., nanomembranes) alters the electronic characteristics of such membranes relative to reference (i.e., no analyte capture) membranes.

In various examples, the detection reagent may comprise a solution of one or more biomolecule conjugates, and the step of adding detection reagent may comprise adding one or more solution of biomolecule conjugates, wherein the biomolecules may comprise any biomolecule (or combination thereof) as selected from those disclosed herein. These biomolecules may be conjugated to an optical detection moiety, wherein these optical detection moieties may include a fluorophore, a chromophore, a fluorescent polymeric nanoparticle, a quantum dot, or an enzyme or other catalytic molecule which exhibits or participates in substrate reduction process (or processes), such that these conjugates possess or yield an emission, a chemiluminescent or absorbance signal at a defined wavelength or range thereof. Further, substrates for enzymatic or catalytic reduction, as well as any required co-reagents for such reduction, may be added sequentially or may be concurrently added with detection reagents. In other examples, the detection reagents may comprise a biomolecule conjugated to a redox agent as disclosed below. Exemplary means for biomolecule conjugation include, but are not limited to, substitution reactions (e.g., nucleophilic attack where a group (e.g., a halogen or other suitable leaving group) is displaced), click reactions (i.e., a 3 + 2 reaction between an azide moiety and alkynyl moiety), other reactions between a nucleophile (e.g., an amine, a thiol, an alkoxide, and the like) and electrophile (e.g., a maleimide, anhydride, epoxide, and the like), and cross-coupling reactions (e.g., a Heck reaction and the like). Non-limiting examples of functional groups and/or reaction partners include silane, amino, carboxyl, thiol/sulfhydryl, isothiocyanate, epoxide, iodo-, alkane, maleimide, succinimidyl, anhydride, mercaptan, hydrazine, N-glycan, or O-glycan, and the like.

In various examples, the addition of detection reagents further comprises adding one or more solution of one or more first non-conjugated detection reagents (i.e., lacking any conjugated optical detection moiety or redox agent) and one or more second conjugated detection reagent (i.e., conjugated to an optical detection moiety or redox agent). For example, the first non-conjugated detection reagents are primary antibodies against multiple analytes, followed by second conjugated detection reagents such as secondary antibodies bearing any of the conjugates disclosed herein, or *S. aureus* Protein A or G bearing any of the conjugates disclosed herein. In these examples, the second conjugated detection reagents bind first non-conjugated detection reagents. Other similar methods are known in the art. In another example, the optical detection modality is surface-enhanced Raman spectroscopy as disclosed in PCT Application No. GB2016/053046 (Pascut et al. "Nanostructured Materials").

In various examples, the step of measuring a signal of one or more detection reagent can comprise an optical modality appropriate for the chromophore, fluorophore, or a chemiluminescent or absorbance signal at a defined wavelength or range thereof. A light source and a detector may be used for excitation and recording of emission, luminescent and/or absorbance signals. Accordingly, a kit of the present disclosure may comprise a light source and a detector, in a variety of fashions including photodiode arrays, charge coupled devices, and other optical sensing techniques for carrying out the optical signal detection methods of the present disclosure.

In an example, wherein nucleic acid of any captured analyte is extracted, the extraction step comprises thermal, chemical, mechanical denaturation, or any combination thereof, that liberates nucleic acids for subsequent application and/or sequencing reactions. The liberated nucleic acids are further captured by DNA and/or RNA oligonucleotide primers that are disposed (e.g., functionalized) onto the wall of a well or reservoir (as another element of the fluidic device), or that were disposed onto the membrane (e.g., nanomembrane) that initially captured the analytes. Accordingly, the kit of the present disclosure may comprise a sonic transducer and/or a heating element for purposes of denaturing captured analytes.

In another example, the functionalized well or reservoir for further capture of extracted nucleic acids comprises another functionalized silicon membrane (e.g., nanomembrane) and/or a functionalized polymeric structure (e.g., SU8 photoresist, polyurethane, poly-dimethyl-siloxane, or cyclic olefin) or an inorganic substrate (e.g., silicon, quartz, or glass wafer). Further, the well or reservoir may comprise one or more membrane, polymeric structure and/or inorganic substrate, where any of these may be selectively functionalized with respect to the others.

In an example, performing a sequencing and/or amplification reaction comprises the addition of sufficient reagents for performing a sequencing and/or amplification reaction, which may comprise solutions of buffers, salts, detergents, deoxynucleotide triphosphate (dNTPs, in native and/or fluorophore-conjugated form), enzymes (e.g., reverse transcriptases, polymerases, and/or ligases), and the like, as required for carrying out the methods. Such reagents may enable RT-PCR, PCR, LAMP, LCR, and/or Taqman^{™} PCR, and the like. Accordingly, a kit of the present disclosure may comprise a heating element for carrying out such amplification and/or sequencing reactions requiring thermal cycling.

In an example, measuring a signal of one or more amplification and/or sequencing reaction products comprises detection of fluorophores incorporated into such reaction products, or release of unique fluorophores as labeled nucleotides are added to amplification or sequencing products, such that such addition releases fluorophores. As another example, a fluorescent or chromophoric dye is added to detect the reaction products, such that the addition of the dye and binding of the dye to the reaction products comprises a fluorescent or colorimetric detection signal. Accordingly, a kit of the present disclosure may comprise a light source and a detector for detection of such reaction products.

In a further example of a method for detecting an analyte of a sample, the optical detection modality comprises a plasmonic-enhanced optical modality (e.g., surface plasmon resonance, plasmon- or surface-enhanced fluorescence, or surface-enhanced Raman spectroscopy). As known to those skilled in the art, incident light may plasmonically excite fluorophores on captured analyte-detection reagent complexes, and thus upon optical interrogation, amplify emission spectra and improve limit of detection and sensitivity. Further, surface plasmon resonance may rely on a shift in refractive index caused by such plasmon excitation, but may be affected by ambient temperature. In the examples of such phenomena disclosed herein, the membranes would be first conformally coated with a noble metal that is plasmonically active (e.g., Au, Pt, Ag, Ir, Rh, and the like) and further derivatized with a spacer molecule with reactive groups for covalent attachment to the metal and to a further biomolecule. Such functionalized membranes would thus be incorporated into fluidic devices and methods for of the present disclosure carried out as disclosed herein. Accordingly, a kit of the present disclosure may include such plasmonically active and functionalized silicon membranes (e.g., nanomembranes), fluidic devices, a light source and a detector, and thermal elements to specify temperature during optical interrogation.

In a further example of a method for detecting an analyte of a sample, electronic interrogations based on flow-through sensor amperometric or impedimetric methods are used (e.g., electrical resistance, impedance spectroscopy, electrochemical redox spectroscopy, and the like). For example, a functionalized membrane (e.g., nanomembrane) may comprise one or more biomolecules that endow the membrane (e.g., nanomembrane) with specific molecular binding capacity. Upon binding of analytes, the pores or slits of such functionalized membranes (e.g., nanomembranes) may be occluded, such that the trans-membrane electrical resistance to an input current increases or is blocked altogether. In an example of such methods, the membrane is derivatized with an antibody that captures an analyte, while in another example of such methods, the membrane may be functionalized with a DNA or RNA oligonucleotide of one or more specified sequence such that it binds sequencing and/or amplification reaction products (e.g., amplicons).

As another example, a function generator is used to generate an input current at high frequency, such that trans-membrane impedance spectra is recorded. The impedance of an interface is generally determined by applying a sinusoidal voltage perturbation, while simultaneously recording the current response. A linear voltage-current response may be obtained by small (e.g., ~10 mV peak to peak). Such voltage-current responses thus provide the related impedance spectra. As another example, a redox agent (e.g., hydrogen peroxide, Prussian Blue, methylene blue, hydroquinone, ferrocene, and the like) may be used as a detection reagent, wherein such detection reagents are added to the appropriate membrane surface and the electrochemical reduction or oxidation of the detection reagents are recorded as impedance spectra. The redox detection reagent should permeate the functionalized membrane (e.g., nanomembrane), and if the membrane is occluded by analyte binding, then the redox detection reagent cannot readily permeate the membrane and will demonstrate reduced redox activity in direct relationship to the concentration of captured analyte. In these examples, the electrical resistance, impedance spectra, and electrochemical redox impedance spectra are compared between a reference membrane (i.e., no capture biomolecule derivatization) and the functionalized membrane (e.g., nanomembrane) used for analyte capture. Accordingly, a kit of the present disclosure may comprise fluidic devices with two or more electrodes, current function generators, and/or algorithms to generate such current traces and process the resultant voltage response signals. In an example of such methods, the membrane may be derivatized with an antibody that captures an analyte, while in another example of such methods, the membrane may be functionalized with a DNA or RNA oligonucleotide of one or more specified sequence such that it binds sequencing and/or amplification reaction products (e.g., amplicons).

As another example of an amperometric electrochemical interrogation, a membrane may be functionalized (e.g., via at one or more covalent bonds) with a conductive coating (e.g., Au or Ag metal, carbon nanotubes, and the like). Such an electrode-acting membrane may be further functionalized with a capture biomolecule to capture an analyte of interest. The electrode-acting membrane may serve as one of the electrodes within the electrochemical system. In such examples, the detection reagent may comprise a second biomolecule suitable for a matched pair, sandwich assay (e.g., a capture antibody and a detection antibody pair wherein each antibody binds different epitopes of the analyte of interest) and the two antibodies and the analyte may form an antibody-analyte sandwich complex. In this example, the capture antibody may be derivatized to the membrane, while the detection antibody may be conjugated to a redox agent (e.g., Prussian blue, methylene blue, hydroquinone, ferrocene, and the like) or an enzyme or molecule capable of reducing a redox agent (e.g., horseradish peroxidase and hydrogen peroxide). An electrochemical redox spectra may be recorded that quantifies the amount of analyte bound within the antibody-analyte sandwich complex on the functionalized membrane in comparison to a reference, non-functionalized membrane, as the redox activity will be in direct relationship to the extent of captured antibody-analyte sandwich complex.

As another example, the conductive coating (e.g., Au or Ag metal, carbon nanotubes, and the like) of the functionalized membrane (e.g., nanomembrane) serves as one of the electrodes of the electrochemical system and the pores or slits of the membrane may serve as selective filters. In solution (rather than on membrane surfaces), sandwich complexes of analytes and two biomolecules may be formed, wherein one biomolecule (e.g., antibody) may be conjugated to a redox agent (e.g., Prussian blue, methylene blue, hydroquinone, ferrocene, and the like) or an enzyme or molecule capable of reducing a redox agent (e.g., horseradish peroxidase and hydrogen peroxide), while the other biomolecule (e.g., antibody) may be conjugated with conductive nanoparticles (e.g., Au or Ag nanoparticles, and the like). If the analyte of interest is present in the sample (e.g., in the presence of the analyte), then a biomolecule-analyte sandwich complex may be formed and may be selectively retained by the pores or slits of the membranes. However, if the analyte is not present in the sample (i.e., in the absence of the analyte), then no biomolecule-analyte sandwich complex will be formed and the capture and detection antibodies, as well as other sample components, will permeate through the membrane upon filtration. The retention of the sandwich complex at the electrode-acting functionalized membrane (e.g., nanomembrane) may therefore allow recording of electrochemical redox spectra. The biomolecules' conjugates are thus brought into close proximity to the electrode-acting membrane, such that the redox agent (i.e., Prussian blue, methylene blue, hydroquinone, ferrocene, hydrogen peroxide) and conductivity enhancing agents (i.e., Au or Ag nanoparticles) are in electrochemical contact with one another. In such examples, the diameter of pores or the width of slits should be specified such that they freely permeate non-complexed biomolecules and sample components, but retain the biomolecule-analyte sandwich complexes.

In the various examples of electrochemical redox spectroscopy methods, the flow-through sensor format comprising functionalized membranes overcome well-known sensitivities of such methods to the presence of electrolytes. For example, most biological samples comprise 1 to 200 mM salt concentration, including all 0.01 mM integer values and ranges therebetween. Such salt concentration may deleteriously affect the detection limits of electrochemical redox spectroscopy methods. However, in flow-through sensor formats, salt concentration may be easily altered subsequent to capture of analytes of interest. For example, analytes of interest are captured with biomolecules within samples comprising typical salt concentration, and following any optional wash steps, are contacted with buffer solutions and/or detection reagent solutions at 1 to 10 µM salt concentration, including all 0.01 µM value integers and ranges therebetween, which may improve the detection limit while maintaining analyte-biomolecular binding. Similarly, pH may be specified to improve the detection limit through contact with buffers and/or detection reagent solutions of specified pH.

For purposes of this disclosure, a biomolecule (e.g., affinity moiety, molecular recognition agent, and the like) possesses specific molecular binding capacity, with a relatively high association rate and low disassociation rate for its cognate target binding molecule or ligand (e.g., analyte). It is generally recognized that for practical purposes, the biomolecule's relatively high association rate and low disassociation rate for its ligand should result in the biomolecule possessing an equilibrium disassociation constant (K_{d}) that are within the range of pM to nM values. The three-dimensional structure of the biomolecule is such that it can form high-affinity interactions upon binding of its ligand through, for example, electrostatic, hydrophobic, ionic, van der Waals, hydrogen-bonding interactions, and the like. For example, the three-dimensional structure of monoclonal, polyclonal or antibody fragments is determined by the amino acid sequence of these proteins, and more particularly, the specific and unique amino acid sequences of the Fv or FaB regions of such proteins determines its affinity for the epitopes of a ligand. As another example, the three-dimensional structure of lectins, and in particular, the specific and unique amino acid sequence of its carbohydrate-binding region determines its affinity for carbohydrate structures of its ligands. As an additional example, the three-dimensional structure of an aptamer is determined by its nucleic acid sequence, such that the resulting three-dimensional structure of the aptamer forms high-affinity binding interactions sites with regions of its ligands. As another example, the nucleic acid sequence of an oligonucleotide determines its sequence-specific binding to complementary nucleic acid sequences through canonical base-pairing interactions. Of course, many other possible biomolecular structural interactions with target ligands are possible and the examples have been provided for exemplary purposes only. In the various embodiments disclosed herein, these exemplary interactions (as well as other possible interactions) describe the manner in which biomolecules interact with target analytes and also describe the manner in which biomolecules interact with detection reagents (e.g., one or more non-conjugated biomolecule (e.g., at least a first and second non-conjugated biomolecule) and one or more conjugated biomolecule (e.g., at least a first and second conjugated biomolecule), as described herein).

For purposes of this disclosure, a ligand represents a portion of an analyte with which a biomolecule interacts. For example, a first ligand could be the epitope of an analyte bound by a monoclonal antibody or the epitopes of an analyte bound by a polyclonal antibody.

The functionalized silicon membrane is a functionalized silicon nanomembrane chosen from a nanoporous silicon nitride membrane, a microporous silicon nitride membrane, a microslit silicon nitride membrane, and a microporous silicon oxide membrane. In an aspect, the present disclosure provides functionalized silicon membranes (e.g., nanomembranes). The functionalized silicon membranes (e.g., nanomembranes) are functionalized with one or more terminal group or moiety (e.g., biomolecule, non-fouling, and/or surface property modifying group). In various examples, a functionalized silicon membrane (e.g., nanomembrane) is made by a method of the present disclosure.

A functionalized silicon membrane (e.g., nanomembrane) has a plurality of functionalizing groups disposed on at least a portion of a surface of a silicon membrane (e.g., nanomembrane). The groups comprise one or more terminal functional groups. The functionalized silicon membranes (e.g., nanomembranes) with one or more terminal functional moieties exhibit one or more desirable properties. Without intending to be bound by any particular theory, it is considered that the terminal functional groups provide one or more desirable properties (e.g., affinity agent, biomolecule, non-fouling group, and/or surface property modifying group, and the like) of a functionalized silicon membrane (e.g., nanomembrane).

The terminal functionalizing groups can be covalently bonded directly to a surface of a functionalized silicon membrane (e.g., nanomembrane) or covalently bonded to a surface of a functionalized silicon membrane (e.g., nanomembrane) via one or more linking groups. For purposes of this disclosure, the terms terminal group and terminal moiety (in both singular and plural forms) are used synonymously.

The functionalization (e.g., individual functionalizing groups) are of appropriate atomic length and molecular size (e.g., molecular volume) such that it does not significantly reduce the permeability of silicon membranes (e.g., nanomembranes). For example, a nanoporous silicon nitride membrane comprises a mean pore diameter of 50 nm. Functionalization of such a membrane with, for example, a three-carbon, five-carbon, or twenty-carbon alkane reduces mean pore diameter by 0.92 nm, 1.5 nm, and 6.2 nm, respectively. In the former two examples, the reduction in mean pore size will not significantly reduce permeability. However, the latter example will significantly reduce permeability (due to a greater than 10% reduction in mean pore diameter). In various examples, the functionalization does not reduce the mean of the longest pore dimension parallel to the longest axis of the pore (e.g., mean pore diameter) of at least a portion of the silicon membrane pores by greater than 10%, greater than 15%, or greater than 20%. Thus, the functionalization of silicon membranes should ideally be of limited atomic length and molecular size in order to not negatively affect membrane permeability. For purposes of this disclosure, a significant reduction in permeability should be considered one that reduces mean pore size by more than 20%.

For purposes of this disclosure, surface density should be considered the number of, for example, surface reactive groups or resultant surface groups on silicon membranes that are covalently bonded to a silicon membrane surface, and thus, should be considered the extent of silicon membranes covered by such groups (i.e., surface coverage extent). The multiple, distinct reactive surface groups may be functionalized using one or more individual chemical processes that form covalently bonded linker and/or terminal groups on silicon membranes. Surface density should be empirically determined buy one of the several metrology methods disclosed herein.

In an example, the surface coverage extent of functionalized surface density of reactive hydroxyl surface groups is 100% (i.e., such groups comprise complete reaction with either the epoxide or the silane functionalization methods described herein). As another example, the surface coverage extent of functionalized surface density of reactive amine surface groups is 100% (i.e., such groups comprise complete reaction with the aldehyde functionalization methods described herein). As another example, the surface coverage extent of functionalized surface density of reactive hydroxyl surface groups is 100% and the surface coverage extent of functionalized surface density of reactive amine surface groups is 100% (i.e., the hydroxyl groups comprise complete reaction with the silane functionalization methods described herein and the amine groups comprises complete reaction with the aldehyde functionalization methods described herein). Without intending to be bound by any particular theory, the extent of chemical activation of surface reactive groups, time, temperature, and concentration of epoxide, silane, and aldehyde reactants may all affect the extent of functionalization surface density. In various examples, the surface coverage extent of functionalized surface density of reactive surface groups (e.g., hydroxyl surface groups, amine groups, silane groups, and the like) is 95, 96, 97, 98, 99, 99.5, 99.9%. In various examples, the surface coverage extent of functionalized surface density is 20% to 100%, including all 0.1 % values and ranges therebetween. In another example, the surface coverage extent of functionalized surface density is 40% to 80%, including all 0.1 % values and ranges therebetween, where such a range provides a useful surface coverage extent. By "useful surface coverage extent," it is meant that the range of surface coverage forms a biomolecule, non-fouling, and/or surface property modifying functionalized membrane (e.g., nanomembrane) for the uses disclosed herein.

The silicon membranes (e.g., nanomembranes) may be nanoporous, microporous, or microslit silicon membranes. The silicon membranes may be referred to as silicon membranes, membranes, or membranes (in both singular and plural forms). Of particular importance to porous or slit membranes, the addition of surface functionalization should ideally be of appropriate atomic length so as to not significantly reduce pore or width sizes, porosity, and/or permeability. Further, such surface functionalization should ideally possess practically no rate of hydrolysis (i.e., comprising covalently stable bonds) within a wide range of chemical and solution environments. In an example, the surface functionalization exhibits no observable no rate of hydrolysis (i.e., comprises covalently stable bonds). The rate of hydrolysis can be determined by methods known in the art. For example, the rate of hydrolysis is determined by a method disclosed herein.

The functionalization should ideally be stable in hydrolytic environments. For example, high (e.g., greater than or equal to 8) or low (e.g., less than or equal to 6) pH, high salt (e.g., greater than or equal to 500 mM total salt), elevated temperature (e.g., greater than or equal to 37 °C), and/or prolonged exposure duration may all promote hydrolysis of functional groups used to derivatize silicon membranes. In examples disclosed herein, amine bonds (i.e., C-N bonds) are preferred due to their increased hydrolytic stability over silane bonds (i.e., Si-O-Si bonds). In further examples disclosed herein, amide-based derivatization of silicon membranes is combined with silane-based derivatization of silicon membranes, such that the combination increases the density and surface coverage, and thus, promotes the hydrolytic stability of both functional derivatives. As is known in the art, silanes are prone to hydrolysis of their Si-O-Si bonds.

In an example disclosed herein, the functionalized silicon membranes are used for sample preparation and the required hydrolytic stability is from several hours to multiple days (e.g., 1-2 hours to 2-3 days, as well as all hour or day 0.1 integer values and ranges therebetween). In another example disclosed herein, the functionalized silicon membranes are used for flow-through sensor applications and the required hydrolytic stability is from several hours to multiple days (e.g., 1-2 hours to 2-3 days, as well as all hour or day 0.1 integer values and ranges therebetween).

For purposes of this disclosure, hydrolytic stability, hydrolytically stable, and non-hydrolyzable should be considered synonymous terms. Such terms refer to the extent of surface modification coverage that resists hydrolysis for the exemplary time-courses described herein. By "resistance" and "stability," it is meant that the extent of surface coverage is unchanged (i.e., no detectable loss of covalently bonded groups) when comparing modified membranes exposed to hydrolytic conditions versus similarly modified membranes not exposed to hydrolyzing conditions, where the comparison to determine changes in extent of surface coverage is performed by one or more of the metrology techniques disclosed herein.

In an example, the silicon membrane is a nanoporous silicon nitride membrane (NPN). Examples of NPN membranes and the fabrication of such membranes are disclosed in U.S. Patent No. 9,789,239 (Striemer et al. "Nanoporous Silicon Nitride Membranes, and Methods for Making and Using Such Membranes").

In another example, the silicon membrane is a microporous silicon nitride membrane (MP SiN). Examples of MP SiN membranes and the fabrication of such membranes are known in art.

In yet another example, the silicon membrane is a microslit silicon nitride membrane (MS SiN). Examples of MS SiN membranes and the fabrication of such membranes are disclosed in U.S. Application No. 62/546,299 (Roussie et al. "Devices, Methods, and Kits for Isolation and Detection of Analytes Using Microslit Filters").

In yet another example, the silicon membrane is a microporous flat tensile silicon oxide membrane (MP SiO₂). Examples of MP SiO₂ membranes and the fabrication of such membranes are disclosed in U.S. Patent No. 9,945,030 (Striemer et al. "Free-Standing Silicon Oxide Membranes, and Methods of Making and Using Same").

Silicon membranes (e.g., nanomembranes) can be chips or dies. In various examples, the silicon membrane (e.g., nanomembrane) structure is a chip or die, where the chip or die is derived from a portion of or the entirety of a silicon wafer substrate. The structures can be monolithic structures, where the chip or die comprises one or more functionalized silicon membrane (e.g., nanomembrane) disposed on a portion or all of the silicon wafer substrate, and further comprising at least one (e.g., one or more) first membrane surface, at least one second membrane surface, one or more aperture, and a plurality of nanopores, micropores, or microslits within the silicon membrane. For purposes of this disclosure, the terms substrate, chip, or die refer to silicon membranes. One or more of these structures, chips, or dies may be incorporated into fluidic devices of the present disclosure.

In the various examples, the silicon membranes (e.g., nanomembranes) comprise a nanopore, a micropore, or a microslit density of 10² to 10¹⁰ pores/mm², including all integer pores/mm² values and ranges therebetween. In the various examples, the silicon membranes comprise a nanopore or a micropore diameter, or a microslit width, of 11 nm to 10 µm, including all µm integer values and ranges therebetween. For NPN membranes, the mean nanopore diameter is, for example, at least 11 nm. The nanopore or a micropore diameter, or the microslit width, is not ≤ 10 nm. The porous or slit layer is disposed on a silicon wafer substrate of <100> or <110> crystal orientation, and further wherein one or more aperture extends through the thickness of the silicon wafer, such that one or more one first membrane surface and at least one second (i.e., opposing) membrane surface are formed by the at least one aperture, and the plurality of nanopores, micropores, or microslits, are fluidically connected to the at least one aperture. The aperture surface comprises internal sidewalls within the substrate, such that each aperture can add significant surface area to the membrane structures. The aperture of the substrate can be formed by standard photolithographic patterning, reactive ion etching of a masking layer, wet chemical through-substrate etching, and other methods known in the art. Through-substrate etching forms apertures connected with each first and each second membrane surface (i.e., formed by the one or more aperture) and the plurality of nanopores, micropores, or microslits, are fluidically connected to the one or more one aperture.

In various examples, an aperture extends through the thickness of the silicon substrate such that a first membrane surface is formed by the aperture, and at least some of the plurality of nanopores, micropores, or microslits are fluidically connected to the aperture at the first membrane surface. In additional examples, one or more additional apertures extend through the thickness of the silicon substrate such that a corresponding one or more additional membrane surfaces are formed by the one or more aperture.

The silicon membranes (e.g., nanomembranes) can have a range of membrane thickness. In various examples, the nanoporous, microporous, or microslit membrane have a thickness between 20 nm and 10 µm, including all integer nm values and ranges therebetween.

The functionalization can comprise various functionalizing groups. In an example, all of the functionalizing groups are the same. In another example, a functionalized silicon membrane (e.g., nanomembrane) comprises a combination of at least two different functionalizing groups. In various examples, the functionalized silicon membrane (e.g., nanomembrane) comprises two or more selectively functionalized membrane surfaces, one or more selectively functionalized aperture, one or more selectively functionalized intra-pore or intra-slit surface, and/or a combination thereof.

In various examples, the silicon membranes (e.g., nanomembranes) can have a range of surface area-to-volume ratios that offer beneficial physical parameters for sample preparation and flow-through sensors. The micron-scale geometry of such structures may promote both solute mass transfer via advantageous diffusion and/or convection of such solutes. In an example, the combined at least one first membrane surface, the at least one second membrane surface, and the plurality of nanopores, micropores, or microslits, further comprises a total surface area-to-volume ratio of 3:1 to 50:1, including all integer ratio value and ranges therebetween. In another example, the combined at least one second membrane surface, the one or more aperture, and the plurality of nanopores, micropores, or microslits, further comprises a total surface area-to-volume ratio of 2:1 to 25:1, including all integer ratio value and ranges therebetween.

The functionalization can comprise various functionalizing groups. In an example, all of the functionalizing groups are the same; e.g., biomolecules. In another example, a functionalized silicon membrane comprises a combination of at least two different functionalizing groups. For example, the functionalization is one or more biomolecule and one or more non-fouling and/or surface property modifying groups. Examples of functionalizing groups are described herein.

The functionalized silicon membranes (e.g., nanomembranes) can be made by methods of functionalizing a silicon membrane described herein. The methods are based on reaction of a reactive surface group on a surface of silicon membrane (e.g., a substrate surface group) with a functional group on a functionalizing group precursor compound. In various preferred examples, the terminal group is one or more biomolecules. Other terminal groups (e.g., non-fouling and/or surface property modifying) may be combined with biomolecule terminal groups.

In various examples, the disclosure describes covalent reaction chemistries for the modification of silicon membranes (e.g., nanomembranes). The functionalization may be terminated with biomolecule, non-fouling, and/or surface property modifying groups. The functionalization may also be referred to as modification or as derivatization.

In an example, the methods disclosed herein for functionalizing silicon membranes (e.g., nanomembranes) comprise one or more selective chemistries which react with unique classes of functional groups of the silicon membranes (e.g., substrate surface groups). Thus, one selective chemistry may be used to functionalize a first substrate surface group, while a second selective chemistry may be used to functionalize a second substrate functional group, and the one or more selective chemistries may comprise distinct bonds linking to the silicon membrane substrate. For example, epoxidation or silanization is used to react with substrate surface hydroxyl groups to form Si-O-C or Si-O-Si bonds, respectively. As another example, reductive amidation forms Si-N-C bonds. In such examples, the first instance of "Si" refers to the Si of the silicon membrane, the second instance of "O" or "N" refers to the atom derived from the substrate surface group, and the final instance of "C" or "Si" refers to the atom of the derivatizing molecule.

In various examples, functionalization methods disclosed herein are combined such that a greater extent of surface coverage and surface functionalization is achieved in comparison to use of only one functionalization method. Further, the combined functionalization may rely on amide bonds (which are less prone to hydrolysis) to protect silane bonds (which are more prone to hydrolysis). Thus, the amide bonds may provide a means for greater surface functionalization that can overcome the well-known problem of incomplete surface coverage of silanes (which promotes their hydrolysis and removal from the substrate surface).

In various examples, a method for the functionalization of silicon membranes using covalent reaction chemistries comprise activation or treatment of the membrane surface by solution-phase chemistries, such that reactive surface groups are formed (e.g., substrate surface hydroxyl or amine groups). These substrate surface groups may be reacted with a first molecule (e.g., first compound) comprising one or more first reactive group that selectively reacts with substrate surface groups and one or more second reactive group that reacts with terminal groups; i.e., the first molecule (e.g., first compound) may be a bifunctional molecule (e.g., bifunctional compound). Examples of such first molecules include epihalohydrins, aldehydes, and silanes, and the like. The second reactive group of the first molecules (e.g., first compounds) may be derivatized with one or more terminal groups (e.g., one or more biomolecules only or one or more biomolecules plus any combination of optional non-fouling groups and/or surface modifying groups). Alternatively, the first molecules (e.g., first compounds) may be optionally cross-linked or covalently reacted to one another and then further derivatized with biomolecules and any optional other terminal groups, and thus comprise at least three or more second reactive groups for such cross-linking and further derivatization (e.g., the first molecules (e.g., first compounds) are trifunctional molecules (e.g., trifunctional compounds)). Alternatively, the first molecules (e.g., first compounds) may be further reacted with spacer molecules (e.g., spacer compounds) to, for example, add a spacer (e.g., an aliphatic group) comprising 1-18 carbons, a third reactive group that reacts with the first molecule's reactive groups, and two or more fourth reactive groups that can react with biomolecules, optional other terminal groups, and optional cross-linkers to other spacer molecules (e.g., spacer compounds). Thus, the spacer molecules (e.g., spacer compounds) may be bifunctional or trifunctional molecules (e.g., bifunctional compounds or trifunctional compounds). The second reactive group of first molecules (e.g., first compounds) and the fourth reactive groups of spacer molecules (e.g., spacer compounds) may react with endogenous, derived, or synthetic surface group (or groups) of the biomolecules and optional other terminal groups.

Means for bonding first molecules (e.g., first compound) to terminal groups, first molecules (e.g., first compounds) to second molecules (e.g., second compounds), second molecules (e.g., second compounds) to terminal groups, cross-linking first molecules (e.g., first compounds) to first molecules (e.g., first compounds), and/or cross-linking second molecules (e.g., second compounds) to second molecules (e.g., second compounds) include substitution reactions (e.g., nucleophilic attack where a group (e.g., a halogen or other suitable leaving group) is displaced), click reactions (i.e., a 3 + 2 reaction between an azide moiety and alkynyl moiety), other reactions between a nucleophile (e.g., an amine, a thiol, an alkoxide, and the like) and electrophile (e.g., a maleimide, anhydride, epoxide, and the like), cross-coupling reactions (e.g., a Heck reaction and the like), and other strategies known in the art. For example, spacer groups are present between first molecules (e.g., first compounds) and terminal groups. In such an example, the spacer group (e.g., spacer compound) is covalently bonded to the first molecule (e.g., first compound) using methods described herein or known in the art, and the terminal group is covalently bonded to the spacer molecule (e.g., spacer compound) also using methods described herein or known in the art. Non-limiting examples of functional groups and or reaction partners include silane, amino, carboxyl, thiol/sulfhydryl, isothiocyanate, epoxide, iodo-, alkane, maleimide, succinimidyl, anhydride, mercaptan, hydrazine, N-glycan, or O-glycan, and the like. In an example, these groups are used for bonding first molecules (e.g., first compounds) to terminal groups, first molecules (e.g., first compounds) to spacer molecules (e.g., spacer compounds), spacer molecules (e.g., spacer compounds) to terminal groups, cross-linking first molecules (e.g., first compounds) to first molecules (e.g., first compounds), and/or cross-linking spacer molecules (e.g., spacer compounds) to spacer molecules (e.g., spacer compounds).

In other examples, the spacer molecules (e.g., spacer compounds), as well as the derivatized or synthetic reactive groups of terminal groups, further comprise a liable bond, wherein the liable bond is readily broken upon a triggering event (e.g., UV irradiation, chemical reaction, and the like).

In various examples, the functionalization of silicon membranes modifies the membrane surface properties for particular applications. In various examples, the functionalization of silicon membranes comprise derivatization with one or more biomolecules that endows the membrane with specific molecular binding capacity. In an example, the terminal group is one or more of the biomolecules disclosed herein, such that the biomolecule-modified membrane possesses specific molecular binding capacity for an analyte of interest.

As an example of functionalization of a silicon membrane with a biomolecule, a membrane is chemically oxidized, reacted with epichlorohydrin, and then reacted with a monoclonal antibody solution to provide a functionalized silicon membrane. As another example, a membrane is chemically oxidized, reacted with epichlorohydrin, and then reacted with an amine-terminated DNA oligonucleotide to provide a functionalized silicon membrane. As another example, a membrane is treated with hydrofluoric acid (HF), reacted with glutaraldehyde, and then reacted with a polyclonal antibody solution to provide a functionalized silicon membrane. In all such examples, the terminal group comprises one or more biomolecules. In these examples, use of any required acid/base catalyst or reductive amination agent is assumed. Of course, many other examples are possible.

In various examples, the terminal moieties are a mixture of biomolecules and additional optional terminal groups, wherein the optional terminal groups include, for example, non-fouling and surface property modifying groups. In other examples, the terminal moieties are a mixture of additional optional terminal groups (e.g., non-fouling and surface property modifying groups) and therefore lack any terminal biomolecules. In other examples, the terminal groups are only biomolecules, only non-fouling groups, or surface property modifying groups.

In various examples, the combined optional non-fouling groups and/or surface property modifying groups promote the binding of analytes by concurrently derivatized biomolecules. For example, a non-fouling group is used to promote non-specific binding, a positively charged group is used to promote negatively charged analytes (e.g., DNA or RNA), while a specified oligonucleotide sequence biomolecule derivative may be used to capture a specific analyte nucleic acid sequence. As another example, a PEG group is used to promote surface wetting and to disrupt non-specific fouling, while multiple polyclonal antibody derivatives is used to capture multiple soluble protein analytes. As another example, ethanolamine is used as a non-fouling group, which because of its small molecular size does not contribute significant steric hindrance, while a DNA aptamer is used to capture a small molecule analyte species (e.g., prescribed or illicit pharmacologically active substance). Other possible examples are known in the art. In other examples wherein only one of non-fouling or surface modifying groups are used for functionalization, such groups endow the functionalized membranes with the properties described herein, lacking the properties of any functionalized biomolecules. In other examples, wherein a mixture of non-fouling and surface property modifying groups are used for functionalization, the combined properties of such a mixture endowed the functionalized membrane with the properties of such functionalizing groups. As one example, a non-fouling group such as ethanolamine or PEG-amine may promote membrane wetting and permeation of sample solution solutes, thereby promoting the contacting and washing steps of the methods disclosed herein. Such promotion may offer beneficial performance properties (e.g., removal of matrix interferent factors).

In further examples, the optional terminal group is a group that promotes non-fouling of the membrane by maintaining a hydration layer (e.g., hydroxyl groups or zwitterionic groups) or by a hydrophobic surface (e.g., per fluorinated groups), wherein either terminal groups prevent non-specific absorption of sample components. Further, the chemical properties of the hydration layer may reduce surface tension, thus promoting the wetting ability of functionalized membranes.

As an example of functionalization of a silicon membrane with a non-fouling terminal group, a membrane is chemically oxidized, reacted with epichlorohydrin, and then reacted with ethanolamine to provide a functionalized silicon membrane. As another example, a membrane is chemically oxidized, reacted with epichlorohydrin, and then reacted with amine-polyethyleneglycol (PEG) to provide a functionalized silicon membrane. As another example, a membrane is HF treated and then reacted with glyceraldehyde to provide a functionalized silicon membrane. As another example, a membrane is HF treated, reacted with glutaraldehyde, and then reacted with ethanolamine to provide a functionalized silicon membrane. In all such examples, the terminal group comprises one or more hydroxyl groups. In these examples, use of any required acid/base catalyst or reductive amination agent is assumed. Of course, many other examples are possible and these examples are understood to be performed as optional combinations with any preceding functionalization with biomolecules.

In an example, the optional non-fouling and/or surface property modifying group has a range of linear or branched groups. Such linear or branch groups (e.g., aliphatic groups) are homogenous (e.g., containing only carbon and hydrogen) or heterogeneous (e.g., containing carbon, hydrogen, and other heteroatoms (e.g., oxygen, sulfur, nitrogen, and the like)) in composition and structural arrangement, and comprises, for example, one or more linear or branch chains (e.g., aliphatic chains). Further, such non-fouling groups may be terminated or substituted with one or more functional groups that endow non-fouling properties (e.g., hydroxyl groups, zwitterions, hydrophobic, and the like) and should not decrease mean pore diameter or slit width by more than 10% (e.g., for every 50 nm of pore diameter or slit width, the linear or branched aliphatic (e.g., alkyl) chains should be less than 20 carbons in length). Non-limiting examples of non-fouling groups include ethanolamine, ethylene and polyethylene glycols and co-polymers thereof, vinyl alcohols or pyridines and polymers thereof, perfluorinated or other terminal fluorine presenting groups and polymers thereof, and the like. Additional non-limiting examples of non-fouling groups include sulfobetaine and analogs and derivatives thereof, Fmoc-lysine, hydroxylamine-O-sulfonic acid, 3-(amidinothio)-1-propanesulfonic acid, 6-carbon to 8-carbon long terminal aldehydes with heavily fluorinated aliphatic (e.g,. alkyl) chains, or perfluorooctanesulfonamide. Fmoc-lysine comprises a fluorenylmethyloxycarbonyl (i.e., Fmoc) protective group at the C1 (alpha) position amine such that reaction to the modified reactive surface groups may occur at the C5 (epsilon) amine group of lysine (e.g., Fmoc subsequently deprotected in N, N-dimethylformamide). Another example zwitterionic terminal group may be H₂N-Lys-Glu-Lys-COOH tripeptide (where the C5 (epsilon) lysine side-chains and C-terminus are functionalized with protecting groups) as a larger zwitterion and hydrogen bonding moiety.

In other examples, the optional terminal group is also a surface property modifying group, such as a charged, non-polar, or amphiphilic moiety, such that the functionalization of silicon membranes with such terminal groups forms a coating wherein the surface properties of the silicon membrane correspond to those of these additional terminal moiety examples. These additional terminal moieties can be linear, branched, or possess one or more charged, non-polar, or amphiphilic groups. Examples of such groups include, but are not limited to, linear and branched aliphatic groups (e.g., alkyl, alkenyl, and the like), primary, secondary and tertiary amines having various aliphatic linear or branched groups covalently bonded thereto, carboxylates or sulfonates having various aliphatic linear or branched groups covalently bonded thereto, amino acids such as alanine, leucine, isoleucine, valine, histidine, arginine, lysine, glutamate, aspartate, and the like.

For the purposes of this disclosure, the terms "terminal groups" or "terminal moieties" can refer to such groups that are derived from listed examples. For example, where ethanolamine is referred to as a terminal group, the terminal group can also be referred to as an ethoxyaminyl group or an aminoethoxyl group. Additionally, "terminal group" or "terminal moiety" is synonymous with "terminal moiety forming molecule."

In various examples, performing any of the reactions disclosed herein comprises contacting the membrane with either solution-phase and/or gas-phase reactant molecules, solutions comprising one or more reactants, or any combinations thereof.

The activation or treatment of the membrane surface by solution-phase chemistries, where reactive surface groups are formed, may be selected such that they are compatible with one or both silicon nitride (SiN) and/or silicon oxide (SiO₂) membranes, as disclosed herein.

In an example, the functionalization methods are performed selectively, such that the entirety of a silicon membrane surface on at least two (e.g., both) of its sides are modified. In another example, only one of the membrane's surfaces is selectively modified, while the opposing membrane surface remains unmodified. Further, the nanoporous, microporous, or microslit features of the membranes can be selectively functionalized within their intra-pore or intra-slit surfaces (e.g., the internal surface of a cylindrical nanopore and a micropore or the internal walls of a cubic prism microslit), while any other surface of the membrane remains unmodified or is selectively modified on one or more such surfaces. As a further alternative, the surface walls of the substrate aperture are selectively modified, while the other features of the membranes remain unmodified.

As an example, any surface, pore, or slit feature is selectively masked such that the masking prevents functionalization, while unmasked surfaces are functionalized. For example, the masking comprises use of a photoresist, where the photoresist is disposed onto the first membrane surface of a microporous or microslit membrane, such that any pore or slit features are not masked (e.g., the porous or slit features remain open and are not disposed by these coatings on their intra-pore or intra-slit surfaces). Subsequent to the disposition of the photoresist, any one of the functionalization methods disclosed herein may be used to modify the intra-pore or intra-slit surfaces, followed by removal of the photoresist in an appropriate solvent (e.g., acetone, developer solution, or toluene). The functionalization method would be selective for the unmasked membrane features such that it does not modify the photoresist. In an example, if the functionalization method should happen to modify the photoresist, such modified photoresist would be removed post-functionalization to exposed an unmodified first membrane surface. Further, the photoresist can be selectively removed without disrupting the functionalized surface, pore or slit. Of course, other possible combinations of selective masking and/or functionalization may be carried out with any degree of iteration of surface, pore, and/or slit, and the above example has been provided for exemplary purposes only.

In other examples, the functionalized membrane is further coated with a polymer that is known to bind biomolecules. Such a polymer is further enhance the extent of sample molecules absorbed and thus able to be detected and/or quantified. For example, any membrane and/or aperture surfaces may be coated with nitrocellulose or polyvinylidene difluoride (PVDF) to absorb proteins from a biological sample, and the absorbed proteins assayed by any of the methods disclosed herein. Nitrocellulose and PVDF may be disposed by any methods known to those skilled in the art (e.g., spin-coating, microstamping, contact transfer, bulk solution techniques, and the like).

The method includes functionalizing a silicon membrane, which comprises: contacting a membrane with a chemical oxidation solution; contacting said membrane with gas-phase epihalohydrin molecules; contacting said membrane with solution-phase acid or base catalysts; and contacting said membrane with solution-phase biomolecules.

The chemical oxidation solution may comprise a solution of 80% w/v sulfuric acid (H₂SO₄) and 30% v/v hydrogen peroxide (H₂O₂), at a mixed ratio, respectively, of 3:1 to 20:1 (or any values therebetween). Such a mixed solution may be referred to as piranha solution. Alternatively, the chemical oxidation solution may comprise an aqueous solution of deionized water, 29% w/v ammonium hydroxide (NH₄OH), and 30% v/v (H₂O₂, at a mixed ratio, respectively, of 5:1:1 to 8:0.5:1, including all 0.1 ratio values and ranges therebetween. Such a solution may be referred to as RCA SC1 solution. Such chemical oxidation solutions likely form hydroxyl surface groups on SiN and SiO₂ membranes (i.e., Si-OH bonds). Contact with the chemical oxidation solution may be performed at a range of temperature and time duration. For example, contact with the solution may be from 25 ° to 150 °C, including all 0.1 °C and ranges therebetween. The time duration may be from 1 to 20 minutes, including all 0.01 minute values and ranges therebetween. Concentration of any solution component, temperature, and time duration are likely to affect the extent of surface hydroxyl group formation.

The epihalohydrin molecules (i.e., epihalohydrins) may comprise epichlorohydrin or epibromohydrin molecules. The epoxide group of such epihalohydrins may react with the hydroxyl groups of the chemically oxidized membrane, the reaction mechanism of which is known to those skilled in the art. Gaseous epihalohydrin may be formed at a range of vapor pressure and/or temperature. For example, the vapor pressure is 1.3 to 2666.5 Pascal, including any 0.01 Pascal value and range therebetween. The temperature may be 25 ° to 100 °C, including all 0.1 °C and ranges therebetween. Contact of the membrane with the gaseous epihalohydrin may also be performed at a range of time duration; e.g., from 1 minute to 16 hours, including all 0.01 minute values and ranges therebetween. Vapor pressure, temperature, and time duration may likely affect the extent to which the membrane is derivatized by the epihalohydrin.

The solution-phase acid or base catalysts may comprise an aqueous solution of a Lewis acid or base at a range of concentration and may promote the re-closure of the epoxide ring and removal of the halogen leaving group For example, the acid or base catalyst may comprise deionized water, 0.01% to 10% v/v hydrochloric acid (HCl), including all 0.1% values and ranges therebetween, 0.01% to 10% v/v sodium hydroxide (NaOH) or potassium hydroxide (KOH), including all 0.01% values and ranges therebetween., and the like. The acid or base catalysis may comprise a range of temperature and time duration. For example, the temperature is from 25 ° to 100 °C, including all 0.1 °C values and ranges therebetween, and the time duration may be from 1 minute to 60 minutes, including all 0.01 minute values and ranges therebetween. Such catalysts are likely to promote the removal of the halogen leaving group and re-closing of the epoxide ring, as known to those skilled in the art.

In some examples, a solution-phase or gas-phase spacer molecule is reacted with the epihalohydrin-reacted membrane prior to reacting said membrane with biomolecules. The spacer molecule (e.g., spacer compound) may comprise one or more amine group that reacts with the epoxide functional group of the treated membrane and one or more reactive group that reacts with one or more biomolecules. In an example, the spacer molecule (e.g., spacer compound) is glutaraldehyde, but many other possible spacer molecules (e.g., spacer compound) could be used. Examples of other spacer molecules (e.g., spacer compounds) include bifunctional molecules, wherein one of the at least functional groups is an epoxide, acyl-azide, succinyl ester, akyl-halide, anhydride, isothiocyanate, or maleidmide, with an aliphatic chain of at least three carbons separating the bifunctional groups.

In another example, a method for functionalizing a silicon membrane comprises: contacting a membrane with a chemical oxide etchant solution; contacting the membrane with solution-phase or gas-phase aldehyde molecules; contacting the membrane with solution-phase biomolecules; and contacting the membrane with solution-phase reductive amination agents.

The chemical oxide etchant solution may comprise an aqueous solution of hydrofluoric acid (HF) or buffered-oxide etchant (BOE), either of which selectively etches native surface SiO₂ on SiN and further forms surface amine groups (e.g., Si-NH₂). The aqueous solution of HF may comprise a range of concentration; e.g., 48% v/v HF may be diluted in deionized water to 0.1% to 10%, including all 0.01% values and ranges therebetween. Alternatively, BOE solutions may comprise a solution of deionized water, 40% v/v ammonium fluoride (NH₄F) and 48% v/v HF, at a mixed ratio, respectively, of 5:1:1 to 50:1:1, including all ratio values and ranges therebetween. As appreciated by those skilled in the art, such chemical oxide etchants would be incompatible with SiO₂ membranes, and thus, this exemplary functionalization method is intended for SiN membranes. Contact with the chemical oxide etchant solution may be performed at a range of temperature and time duration. For example, contact with the solution is at a temperature from 25 ° to 60 °C, including all 0.1 °C and ranges therebetween. The time duration may be from 30 seconds to 3 minutes, including all 0.01 minute values and ranges therebetween. Concentration of solution components, temperature, and time duration are likely to promote extent of native oxide removal and amine group formation.

The aldehyde molecules (e.g., aldehyde compounds, such as solution or gas-phase aldehyde compounds) may comprise linear or branched aliphatic (e.g., alkyl) groups with 1-18 carbons with any degree of branching, and one or more terminal aldehyde groups (e.g., glutaraldehyde). Reaction of the aldehyde groups with surface amine groups likely follows a reaction mechanism well-known to those skilled in the art (e.g., a reaction of the aldehyde and amine likely produces a Schiff base imine). The imine be further reduced in order to promote its hydrolytic stability in the form of an amine that is linked to the membrane surface (i.e., Si-N-C bonds).

The gas-phase aldehydes may be formed at a range of vapor pressure and/or temperature. In various examples, the vapor pressure is 1.3 to 2666.5 Pascal, including all 0.1 Pascal values and ranges therebetween, and/or the temperature may be 25 ° to 200 °C, including all 0.1 °C values and ranges therebetween. Contact of the membrane with solution-phase aldehydes may comprise a range of concentration and/or temperature. For example, the aldehyde concentration is be 1 µM to 10 M, including all 0.01 integer µM values and ranges therebetween, and/or the temperature is from 25 ° to 100 °C, including all 0.1 °C values and ranges therebetween. For both solution-phase and gas-phase aldehydes, the contact may be performed at a range of time duration (e.g., from 1 minute to 16 hours, including all 0.01 minute values and ranges therebetween). Vapor pressure, concentration, temperature, and time duration may likely affect the extent to which the membrane is derivatized by the aldehyde.

The contact with the aldehydes may further comprise use of a dehydrating agent; e.g., a molecular sieve, magnesium sulfate, tris(2,2,2-trifluoroethyl)borate, or titanium ethoxide, and the like. Such dehydrating agents may promote formation of the Schiff base amine, as the equilibrium of amine formation from aldehydes and amines may favor the carbonyl compound and the amine reactants.

The solution-phase reductive amination agents may comprise an aqueous solution of, for example, sodium borohydride (NaBH₄), sodium cyanoborohydride (NaBH₃CN), or sodium triacetoxyborohydride (NaBH(OCOCH₃)₃), and the like. Such agents may be at a range of concentration; e.g., 1 µm to 1 mM, including all 0.01 µM values and ranges therebetween. The reductive amination may be performed at a range of temperature (e.g., 25 ° to 100 °C, including all 0.1 °C values and ranges therebetween) and/or for a range of time duration (e.g., 1 minute to 60 minutes, including all 0.01 minute values and ranges therebetween)

In a further example, a method disclosed herein is combined with well-known silane functionalization methods, such that the combination improves the density of surface functionalization coverage, and therefore improve the hydrolytic stability of the silane-functionalized surface. Such combined functionalization methods may rely upon selective mechanisms and reactive groups for the one or more functionalization methods. For example, the method disclosed herein for amine group functionalization (e.g., aldehyde reactions) are combined with a method for hydroxyl group functionalization (e.g., silane reactions).

In various examples of the combined functionalization method, the molecular size (e.g., molecular volume) of the aldehyde derivative should be specified such that it does not sterically hinder further surface derivatization with the silane derivative. Further, the size of the silane derivative should ideally be specified such that it is not sterically hindered by the preceding derivatization of the membrane with the aldehyde derivative. Thus, the number of atoms (e.g., number of atoms in an aliphatic group (e.g., methylene groups and the like) in a chain), number of reactive functional groups, and/or extent of chain branching may be specified for both the aldehyde and silane derivatives. For example, the aldehyde comprises two reactive groups and a five-carbon aliphatic (e.g., alkyl) chain, while the silane comprises one reactive group, two leaving groups, and a two-carbon aliphatic (e.g., alkyl) chain that further branches at the terminal carbon with two methyl groups. Other combinations of which are known in the art. In an example, the silicon membrane is not functionalized solely with a silane.

In a further example, a method for a combined functionalization of a silicon membrane comprises: contacting a membrane with a chemical oxide etchant solution; contacting the membrane with solution-phase or gas-phase aldehyde molecules; contacting the membrane with solution-phase reductive amination agents; contacting the membrane with solution-phase or gas-phase silane molecules; and contacting the membrane with solution-phase biomolecules.

In examples of a combined functionalization, the method for contacting a membrane with solution-phase and/or gas-phase chemical oxide etchants, aldehydes, and reductive amination agents comprises the steps disclosed herein for such contacting steps when only aldehyde-based functionalization is been performed.

The solution-phase or gas-phase silane molecules may further comprise a first reactive group that reacts with the substrate surface hydroxyl groups and a second reactive group that reacts with biomolecules and optional terminal moieties as disclosed herein, such silanes acting as spacer molecules and may include, for example, ethyl 3-[chloro(dimethyl)silyl]acrylate or (3-glycidoxypropyl)trimethoxysilane, and the like.

The gas-phase silane molecules may be formed at a range of vapor pressure and/or temperature. In various examples, the vapor pressure is 1.3 to 2666.5 Pascal, including all 0.1 Pascal values and ranges therebetween and/or the temperature is 25 ° to 200 °C, including all 0.1 °C values and ranges therebetween. Contact of the membrane with solution-phase silane molecules may comprise a range of concentration and/or temperature. For example, the silane molecule concentration is 1 µM to 10 mM, including all 0.01 µM values and ranges therebetween and/or the temperature is from 25 ° to 100 °C, including all 0.1 °C values and ranges therebetween. For both solution-phase and gas-phase silane molecules, the contact may be performed at a range of time duration; e.g., from 1 minute to 16 hours, including all integer minute values and ranges therebetween. Without intending to be bound by any particular theory, vapor pressure, concentration, temperature, and time duration may likely affect the extent to which the membrane is derivatized by the silane.

In some examples of the combined functionalization method, an optional oxidation step precedes contact with the silane(s). For example, a rinse in deionized water for 1 to 10 minutes at 25 ° to 100 °C, including all 0.1 °C values and ranges therebetween, is used to re-form substrate surface hydroxyl groups. Such hydroxyl groups may be removed by oxide etchants, and thus, increasing their density may improve the extent to which silanes derivatize the membranes in subsequent reactions.

In an example, a further method for functionalizing a silicon membrane comprises: performing a conformal metal coating on the membrane; contacting the membrane with either a solution-phase or a gas-phase spacer molecule; and contacting the membrane with solution-phase biomolecules.

In an example, the conformal metal coating comprises Au deposited by one of electron-beam evaporation, thermal evaporation or physical vapor deposition. The time duration may comprise a range of 10 seconds to two minutes, including all 0.01 second values and ranges therebetween. The time duration may affect the thickness of the deposited conformal Au coating and should be specified such that the thickness does not occlude pores or slits and thus reduce the membrane's permeability.

In an example, the spacer molecule (e.g., spacer compound) comprise a bifunctional molecule (e.g., bifunctional compound), wherein the molecule (e.g., compound) comprises one or more sulfhydryl group and one or more reactive group that reacts with the biomolecules, such that the sulfhydryl group reacts with Au and the other reactive group reacts with the biomolecules.

In the various examples, contact with the solution-phase and gas-phase reactants is sequentially performed or concurrently performed in any combination of the various steps. The steps may be performed in suitable reaction vessels for such reactions (e.g., specified volume and surface properties, temperature control, fluidic valves for adding and removing reactants, pumps for controlling vapor pressure, and the like). Further, any of the sequentially and/or concurrently performed steps may be carried out in one common vessel (to which various reactants are added and removed as required for carrying out the method) or in a series of independent vessels (to which various reactants are added and removed and silicon membranes transferred between such vessels, to carry out the method).

In the various examples, optional rinsing or cleaning steps precede or follow any of the steps disclosed herein. Such rinsing or cleaning steps may be performed to remove any chemisorbed or physisorbed reactants and/or reaction products, and the like. The rinsing and cleaning may be carried out with a variety of polar or non-polar solutions (e.g., water, acetone, toluene, dichloromethane, hexane, ethanol, methanol, and the like). Further, an optional drying step may precede or follow any of the steps disclosed herein. For example, membranes may be functionalized by a method of the present disclosure, optionally rinsed in ultra-pure water, then dried under a stream of anhydrous nitrogen gas. Of course, many other possibilities for such optional rinsing, cleaning, and drying steps are possible.

In the various steps of the methods disclosed herein, the reaction is monitored by one or more suitable metrology methods and/or techniques (e.g., variable angle ellipsometry, x-ray photoelectron spectroscopy (XPS), low-energy ion scattering (LEIS), atomic force microscopy (AFM), scanning or transmission electron microscopy (SEM or TEM), contact angle goniometry, infrared absorption spectroscopy (IRAS), and the like).

In the various examples of the membrane functionalization methods disclosed herein, the solution-phase biomolecules (e.g., molecular recognition agents, affinity moieties, and the like) comprise a solution of one or more of the biomolecules selected from the biomolecules as disclosed herein. For example, the epoxide groups of epihalhydrin-derivatized membranes react with amine groups of biomolecules. As another example, the aldehyde groups of glutaraldehyde-derivatized membranes react with biomolecule amine groups. As another example, isothiocyanate groups of silane-derivatized membranes react with amine groups of biomolecules. Of course, other reactions and reactive group combinations are possible.

The solution-phase biomolecules may comprise contacting the epihalohydrin-, aldehyde-, and/or silane-derivatized membranes at a range of temperature; e.g., 25 ° to 40 °C, including all 0.1 °C values and ranges therebetween, a range of concentration; e.g. 0.01% to 10% w/v, including all 0.01 percent values and ranges therebetween, or a range of time duration; e.g., 1 to 16 hours, including all 0.01 hour values and ranges therebetween. Concentration, time duration, and temperature may affect the density at which biomolecules derivatize membranes.

In various examples, the derivatizations of membranes with biomolecules further comprise a range of techniques for depositing biomolecules onto membranes for such reactions. For example, single or multiple unique biomolecule solutions are discretely or continuously disposed onto multiple membrane and/or aperture surfaces using multiple disposition, photolithographic, microstamping, contact transfer, bulk solution techniques, or any combination thereof. In various examples, the disposition of biomolecule solutions comprise using a discrete liquid dispensing technique, such that biomolecule solution droplet volumes of 10 pL to 10 µL, including all 0.01 pL values and ranges therebetween, are disposed as a circular feature of diameter corresponding to dispensed volume and surface properties of the membrane and/or aperture surfaces. In other examples, the disposition comprises continuous disposition of biomolecule solution droplets onto any membrane surface and/or aperture surface, such that a line of length equal to or less than the total width of the membrane and/or aperture surface is disposed with biomolecule solution.

In various examples, forming biomolecule solution droplets further comprises either piezoelectric, positive pressure, or air displacement pipetting techniques using electromechanical or pneumatic actuation, wherein the actuation comprises manual actuation by a trained operator or comprises semi-autonomous or fully autonomous actuation by programmable logic controllers. In the various examples, a biomolecule solution comprises one biomolecule or comprises multiple biomolecules.

In other examples, at least one first membrane surface, at least one second membrane surface, and/or aperture surface can be uniquely or similarly disposed with one or more biomolecule solution or solutions, with any degree of repetition and iteration. As another example, one or more biomolecule solutions are disposed as continuous lines onto at least one first membrane surface, at least one second membrane surface, and/or aperture surface, such that multiple such surfaces are successively disposed with any degree of repetition and iteration. Any degree of repetition and iteration refers to droplets that are disposed on a surface such that all or substantially all of the surface area has droplets disposed thereon. Degree of repetition and iteration may further refer to a pattern disposed on a surface. Successively disposed refers to when two or more surfaces have the same pattern disposed thereon (e.g., five surfaces have the same pattern). As another example, multiple discrete biomolecule solution droplets are disposed on multiple first membranes, such that each discrete biomolecule solution droplet comprises a biomolecule to capture one analyte species. This example would permit multiplex analyte detection. As another example, multiple biomolecule solutions are disposed into multiple second membrane and aperture surfaces, such that each second membrane and aperture surface is uniquely and continuously disposed with one biomolecule solution, wherein each biomolecule solution comprises a biomolecule to capture one analyte species. This example would enable multiplex analyte detection. As another example, multiple biomolecule solutions are disposed into multiple second membrane and aperture surfaces, such that each second membrane and aperture surface is uniquely and continuously disposed with one biomolecule solution, wherein each biomolecule solution comprises a mixture of biomolecule to capture one analyte species and one DNA/RNA oligonucleotide primer for amplification or sequencing of nucleic acids extracted from the captured analyte. This example would enable multiplex genomic assays.

In various examples, the biomolecule and any optional passivation (i.e., blocking) agents are suspended in an aqueous buffer of pH between 4.0 and 10.0 and where the buffer comprises total dissolved salt of any included species between 1 nM and 1 M, including all 0.01 nM values and ranges therebetween. The solutions of biomolecules and/or passivation agents can be suspended in preparations of animal or human whole blood, or fractions thereof. Such passivation or blocking agents are used to reduce non-specific absorption of sample components. Such blocking agents can further be biomolecules or synthetic molecules, or combinations thereof.

In various examples, a stabilizer reagent are additionally deposited onto the membrane and/or aperture surface to maintain surface properties conveyed previously by any functionalization or disposition method. The stabilizer reagent may contain one or more nonreducing sugars, polyols, surfactants, and wetting agents and may also contain one or more species of isothiazolinones, azides, other synthetic biocides, and the like. The stabilizer reagent is removed after co-incubation with the membrane and/or aperture surface for a period of 0.1 to 4 hours, including all 0.01 hour values and ranges therebetween, using vacuum or manual aspiration, leaving a residual film thickness of between 10-1000 micron, including all 0.01 micron value and range therebetween, and further, the stabilizer may be dried to residual water content of between 0.01-5%, including all 0.01 percent value and range therebetween, via freeze-drying, vacuum desiccation, or thermal processing, and combinations thereof. The optional blocking and/or stabilizer solutions may be disposed onto any membrane and/or aperture surface using any of the methods disclosed herein for disposition of biomolecule solutions.

In an aspect, the present disclosure describes fluidic devices incorporating one or more functionalized silicon membrane and uses of such fluidic devices.

In various examples, the fluidic devices comprise filtration devices for analyte capture (i.e., sample preparation) and analyte capture and detection (i.e., flow-through sensors for diagnostic assays and/or liquid biopsy assays).

In various examples, a fluidic device comprises at least one functionalized silicon membrane (e.g., nanomembrane), and further comprises a plurality of fluidic channels or chambers (e.g., a first fluidic channel or chamber, a second fluidic channel or chamber, etc.) in fluidic contact with a plurality of membrane surfaces (e.g., a first membrane, a second membrane, etc.), such as, for example, a first fluidic channel or chamber in fluidic contact with a first membrane surface and at least one second fluidic channel or chamber in fluidic contact with the at least one second membrane surface and one or more aperture, and the plurality of fluidic channels and/or chambers (e.g., a first and second fluidic channels and/or chambers) in fluid contact with each other via the aperture and the nanopores, micropores, or microslits, of the membrane.

In various examples, a fluidic devices comprises a first fluidic channel and/or chamber in fluidic contact with the silicon substrate; a second fluidic channel and/or chamber in fluid contact with the membrane (e.g., nanomembrane); and wherein the first fluidic channel and/or chamber is in fluidic communication with the second fluidic channel by way of the aperture and the plurality of nanopores, micropores, or microslits of the membrane. In various examples, a first plurality of fluidic channels and/or chambers are in fluidic contact with a silicon substrate (e.g., silicon wafer); a second plurality of fluidic channels and/or chambers are in fluidic contact with the membrane (e.g., nanomembrane), wherein the first plurality of fluidic channels and/or chambers are in fluidic communication with a second plurality of fluidic channels and/or chambers by way of an aperture and a plurality of nanopores, micropores, or microslits.

In various examples, wherein one or more additional apertures extend through the thickness of the silicon substrate, and wherein the first fluidic channel and/or chamber (or plurality thereof) is further in fluidic communication with the second fluidic channel and/or chamber (or plurality thereof) by way of the one or more additional apertures.

In various examples, a method of performing a filtration comprises: contacting an input sample with a functionalized silicon membrane, where the input sample contacts at least one first membrane surface of a membrane; and permeating a fraction of the input sample to the second (opposing) and aperture surface of the membrane.

In an example, contacting the input sample with the at least one first membrane surface comprises normal or tangential flow relative to the membrane surface, where such flow comprises one of gravity flow, hydrostatic pressure, pumping, vacuum, centrifugation, gas pressurization, or combinations thereof.

In another example, the method further comprises contacting the at least one second membrane surface and/or at least one aperture with an optional second solution during permeation of the permeating fraction of the input sample. For example, the second solution is a buffer solution.

In other examples, contacting the at least one second membrane surface and/or at least one aperture with an optional second solution further comprises flowing the optional second solution parallel with, perpendicular to, or counter to, the flow of the input sample. In this example, permeation of solutes from the input solution to any optional second solution or permeation of solutes from any optional second solution to the input solution may occur. Such permeation may promote transfer of solutes (i.e., analytes of the sample) from the first membrane surface to the second membrane and aperture surfaces, wherein such transfer enhances analyte capture by convective and diffusive mass transfer effects.

In other examples, contact of wash, elution, and other solutions disclosed herein, further comprises contact and permeation as described for input samples.

In an aspect, the present disclosure comprises kits. For example, the kits are kits for carrying out the methods of the present disclosure.

A kit comprises one or more fluidic devices of the present disclosure and instructions for using same (e.g., to carry out a method of the present disclosure).

In various examples, a kit of the present disclosure further comprises instructions, buffers, solutions, reagents, and the like, as required for carrying out the methods of the present disclosure.

In various examples, a kit of the present disclosure further comprises one or more functionalized silicon membranes, one or more well or reservoir, one or more fluidic devices, one or more light source and detector, one or more sonic transducer, one or more heating element, and the like, for carrying out the methods of the present disclosure.

In various examples, a kit of the present disclosure further comprise one or more signal processing algorithm, one or more operating system, and/or one or more programmable user interface, for carrying out the method of the present disclosure.

The steps of the method described in the various embodiments and examples disclosed herein are sufficient to carry out the methods of the present disclosure. Thus, in an embodiment, a method consists essentially of a combination of steps of the methods disclosed herein. In another embodiment, a method consists of such steps.

The following examples are presented to illustrate the present disclosure. They are not intended to limiting in any matter.

### EXAMPLE 1

This example provides a description of preparation and characterization of functionalized of silicon nanomembranes of the present disclosure.

Chemistry Deposition System development and testing. This example describes gaseous phase surface derivatization process for low-stress SiN membrane substrates. Additionally, surface decoration will be monitored by subsequent interaction with reactive species.

Materials. Chemicals used for surface functionalization included 3-(triethoxysilyl)propyl Isocyanate, (+/-) epichlorohydrin, ethanolamine, toluene (Anhydrous), N-propanol, dimethyl sulfoxide (DMSO), and Fluorescein Isocyanate Isomer 1 were used as received from Sigma Aldrich at ASC grade or better. The Figures 1 and 2 shows the relevant chemical structures for surface derivatizing schemes explored in this work.

Experiment Setup. A basic vacuum deposition system was fabricated from off-the-shelf components. Images of the system used are attached for reference. Briefly, a vacuum source (mid-range rotary vane pump) is connected via inline vapor trap (pre-loaded with molecular sieves) to a polycarbonate desiccator dome (Nalgene Inc.). Inside the dome was placed a sample holder (entirely polypropylene construction), elevated ~ 3" from the chamber bottom to promote ideal gas flow to the samples. The dome inlet supplies (vie straight wye) either 0.2 micron filtered atmosphere vent or chemistry vapor generated from a borosilicate glass tube (25 mL capacity). Both inlet types are individually controlled via full-port ball valves. A pressure gauge (VWR brand, NIST traceable) is used to monitor system pressure inline to the chemistry flask. After assembly the system was leak checked and is suitable for maintaining a 8 kPa vacuum for at least 24 hours.

Preparation of Substrates. A 4" SiN coated double-side polished wafer was used as the source material for all experiments. Individual die were cleaved into ~ 1cm² surface are substrates and held in glass dishes until use. All substrates used in deposition experiments were cleaned using a standard Piranha wash (3:1 H₂SO₄:H₂O₂) for 1 hour at RT, then rinsed in excess with nanopure 18.6 MΩ water and used immediately.

Deposition of Epichlorohydrin. Substrates (prepared as above) were placed on the sample holder in the dome, then the system was evacuated of atmosphere to at least 8 kPa. Following which 2 grams of epichlorohydrin was allowed to vaporize from the chemistry flask at RT over a period of 2 hours as follows:
1) Achieve base vacuum pressure
2) Close vacuum source valve
3) Open chemistry valve
4) Wait 1 hour
5) Open vacuum source valve
6) Pump vacuum for 1 hour
7) Purge to atmosphere <10 minutes
During this process all of the liquid chemistry was converted to a vapor at RT conditions. Following deposition substrates were recovered from the sample holder and stored in pre-cleaned glass dishes until use.

Deposition of Isocyanate Silane. A series of substrates were prepared as above and collected in a toluene-cleaned glass dish. To these substrates a solution of 10% 3-(triethoxysilyl)propyl Isocyanate (NCO-silane) in anhydrous toluene was added and allowed to react for 2 hours at room temperate with no agitation. Following deposition sensors were rinsed extensively in the dish with fresh toluene, then transferred to a new toluene-containing dish, further rinsed with 2X fresh toluene fractions, then sonicate for 5 minutes to remove nonspecifically adsorbed silane species. After sonication, the waste toluene was displaced with N-propanol via several successive fraction rinses, then each substrate was rinsed under N-propanol stream and N2 dried. Substrates were collected in a clean glass dish until use.

Verification of Surface Chemistry Reactivity. To verify the surface reactivity of both epoxide and isocyanate derivatized substrates, solutions of ethanolamine and BSA were prepared and allowed to incubate overnight at RT with 250 RPM agitation. Ethanolamine was deposited from a 100 mM solution containing 50 mM borate pH 9.0 whereas BSA was deposited from a 1% solution in PBS pH 7.4. After deposition both solutions were displaced with a wash buffer containing PBS augmented with 0.05% Tween-20 and 5 mM EDTA pH 7.4 (PBS-ET) for 30 minutes at RT with 250 RPM agitation. After washing substrates were individually rinsed under freshly prepared nanopure water stream and N₂ dried.

Fluorescent labeling of surfaces. As a verification of the presence of BSA on each surface type, the surface-bound BSA was fluorescently labeled for later quantitation. A solution of 200 µg/mL FITC isomer 1 was first prepared as a 6 mg/mL solution in DMSO then diluted into 50 mM Sodium Borate pH 9.0. This solution was then applied from bulk to all substrates and allowed to react for 2 hours with 250 RPM agitation. Following deposition, substrates were individually rinsed in borate buffer then nanopure water and finally dried under N₂ stream.

Contact angle measurements. Sessile water contact angle measurements were collected in triplicate per sensor substrate via deposition of a 2 µL droplet of freshly prepared 0.2 µm filtered nanopure water, then imaged use a USB camera and MicroCapture Pro. Images were then analyzed further in image J for sessile contact angles and post processed using Microsoft Excel.

Fluorescent Intensity Measurement. Surface fluorescence profiles were collected for all conditions via mounting of substrates onto a black 384-well plate pre-coated with a 300 µm silicone gasket to prevent motion of substrates during plate manipulation. Fluorescent intensity was collected via an I3 multimode plate reader and SoftMax Pro 6.3 using a 16-point per well scan of each well, where each substrate covers ~ 2.5 wells, yielding at least 32 points per substrate.

The vacuum deposition system fabricated yielded expected performance given the low-cost vacuum pump utilized. Curiously a ~8 kPa vacuum was suitable for vaporizing the chemistry used in this work, likely due to partial pressure combined with continual evacuation of the chamber during dehalogenation through the second hour of the reaction. Initial film deposition performance was only monitored using water contact angle, a limitation of this study. The results for this assessment are presented as Figure 1. As the data shows, native films for both surface functionalities elicited a considerable increase in water contact angle relative to a piranha washed control (<10°, data not shown). Importantly, this increase in hydrophobicity correlates well to the surface terminal groups predicted by the mechanism for both chemistries ('Reaction A', in the attached schemas). While it is difficult to survey the packing density using this method alone, further reaction of the films to amine compounds including ethanolamine and serum albumin yielded an expected change in sessile contact angle. In both cases, ethanolamine coated films yield a substantial reduction in hydrophobicity which correlates well to the addition of the terminal oxygen and secondary amine to the surface. Similarly, coupling serum albumin to each surface chemistry reduced the surface hydrophobicity, though not as significantly as one would expect. This is likely due to the unfolding of the protein during desiccation which exposes the more hydrophobic core of the molecule during contact angel analysis.

As further verification of the presence of covalently tethered serum albumin, the free amines of the protein were further decorated with FITC. This process, after analysis, yielded a considerable increase in MFI for the BSA-treated substrates, with no appreciable MFI change for the ethanolamine passivated substrates relative to the native film controls.

These data demonstrate a system and process has been constructed and tested suitable for the vapor-phase functionalization of SiN surfaces by an epoxide terminal species. Additionally, the silylation of low-stress SiN has been demonstrated using a model amine-reactive alkoxy silane. Both films produced demonstrate sufficient density to affect a water contact angle change and are sufficiently reactive to amine containing compounds by measurement via the former.

### EXAMPLE 2

This example provides a description of preparation and characterization of functionalized of silicon nanomembranes of the present disclosure.

Non-fouling demonstration of Ethanolamine terminated SiN. The following describes the non-fouling potential of ethanolamine derivatized SiN using an assortment of biofluids.

Methods. SiN Preparation. This Example utilized piranha cleaned SiN for all surface derivations. An overview of the functionalization process is provided below.

Substrate Cleaning. An SiN wafer was cleaved into ~ 0.75 cm₂ substrates, then cleaned via a standard 3:1 piranha recipe for 1 hour at RT. Following cleaning, chips were rinsed in bulk and then individually with freshly prepared 0.2 micron filtered 18.6 MΩ water and then dried under N₂ stream.

Epoxide Functionalization. Using the vacuum deposition system (previously described), cleaned SiN die were transferred to the sample holder, then further dehydrated via a 10 min desiccation at 8 kPa. After which 2 grams of (±)-epichlorohydrin (Sigma 481386) was allowed to vaporize into the desiccator dome with the vacuum source isolated for 60 minutes. Following deposition, the chamber was purged to vacuum and allowed to further desiccate for an additional 60 minutes to promote dehalogenation of the surface-bound epichlorohydrin species.

Ethanolamine Deposition. A 10 mM ethanolamine solution was prepared in pH 9.0 Sodium Borate, then exposed to chips previously epoxide-functionalized for 60 minutes at RT in a toluene-cleaned borosilicate glass dish. Following exposure chips were rinsed with NanoPure water extensively and dried under N₂ stream. Contact angle measurements were conducted throughout each step in the above process to ensure consistency with past deposition results.

Biofluid Exposure. After surface treatment, at least 3 chips were exposed to the following conditions: 1% BSA in PBS pH 7.4; 10 % Calf Serum in PBS pH 7.4; and 100% Calf Serum.

Exposure was conducted in pre-cleaned glass dishes and occurred at RT for 24 hours using 250 RPM orbital agitation. As controls, piranha-cleaned and native SiN die were exposed to the identical solutions as above. Following exposure, chips were briefly rinsed in PBS, then NanoPure water, and dried under N₂ stream.

Surface Labeling. To visualize non-specifically adsorbed protein species, all chips were labeled using a 1 µM solution of FITC prepared in pH 8.0 Sodium Borate for 1 hour. Following incubation with the fluorophore, chips were rinsed with NanoPure water and dried under N₂ stream. Dry chips were then collected on a 384-well plate, then read using the well-scan mode of the I3 plated reader at excitation and emission wavelengths for Fluorescein. After which raw MFI was exported to Microsoft Excel for further analysis.

Sessile water contact angle measurements collected through the surface deposition process were consistent with past results for each surface treatment including native SiN (45°±1.8°), piranha cleaned SiN (<5°±2.4°), epichlorohydrin terminated SiN (52°±1.6°), and ethanolamine terminated SiN (22°±2.2°). Surface protein adsorption after 24 hour insult by either purified BSA, dilute serum, or neat serum as monitored by fluorescent labeling by FITC indicated the ethanolamine treatment tends to repel surface fouling for all solutions test (Figure 8). Note, these data are shown as net MFI relative to non-protein exposed die of each surface treatment type. While results are less compelling for BSA treated surfaces, the ethanolamine treated SiN resists ~90% protein adsorption from both neat and dilute serum. This effect is likely due to both the hydrophilicity of the surface treatment which forms a strong hydration layer, prohibiting hydrogen bonding to proteins in solution, as well as the near-zero net charge of the film due to the positively charged secondary amines and negatively charged alcohol groups decorating the solvent-accessible surface.

These data demonstrate the resistance of ethanolamine-treated SiN to biofouling using a limited subset of solution types and exposure modalities. Indirectly, the prolonged non-fouling effects of the ethanolamine treated SiN indicates the linker chemistry is reasonably stable under aqueous buffered conditions for at least 24 hours of continual exposure. Further work is necessary to fully characterize both the reproducibility of surface treatment performance as well as robustness in manufacturing technique.

### EXAMPLE 3

The following example describes uses of the nanomembranes of the present disclosure.

Demonstration of increased analyte capture using a flow-through nanomembrane sensor exposure format relative to conventional normal or sessile target incubation formats currently in use.

Methods. Silicon nitride nanomembranes of either 100 nm thickness with pores of 45 nm average diameter at 20% porosity, or 400 nm thickness with pores of 500 nm average diameter at 20% porosity were utilized for these experiments and processed as follows.

Substrate Cleaning. A membrane-patterned SiN coated wafer was cleaved into 5.4x5.4 mm square substrates, then cleaned via a standard 3:1 piranha recipe (H₂SO₄:H₂O₂) for 30 minutes. Following cleaning, chips were rinsed extensively with freshly prepared 0.2 micron filtered 18.6 MΩ water and then dried under 0.2 µm filtered N₂ stream.

Epoxide Functionalization. Using the vacuum deposition system (previously described), cleaned membranes were transferred to a sample holder, then further dehydrated via a 10 min desiccation at 8 kPa. After which, 1 mL of (±)-epichlorohydrin (Sigma 481386) was allowed to vaporize into the desiccator dome with the vacuum source isolated for 90 minutes. Following deposition, excess chemistry and the surface leaving group was evacuated from the chamber by further desiccation at 8 kPa for 60 minutes. After this dehydration process functionalized nanomembranes were used immediately or stored at RT in clean polystyrene dishes until use.

Study A: Streptavidin Detection via Biotinylated sensors. PEG-Biotin deposition. 400 nm SiN thick Epichlorohydrin treated die were further functionalized via a 1.0 µM solution of Amine-PEG-Biotin (Thermo Scientific #21346) prepared in PBS pH 7.2. All die were immersed in the Amine-PEG-Biotin solution for 60 minutes at RT with gentle orbital agitation. Following deposition, die were rinsed with copious volumes of freshly prepared 0.2 micron filtered 18.6 MΩ water and then dried under 0.2 µm filtered N₂ stream.

Streptavidin detection. Biotinylated die were then assembled into centrifugal spin column devices (examples of which are shown in Figure 12a, b, e, f), creating a fluidically isolated reservoir above the biotinylated membrane. A dilution series of Streptavidin-Alkaline phosphatase conjugate (SA-AP) was prepared in 1% BSA in PBS-ET (1x PBS, .05% Tween 20, 5 mM EDTA), then 500 µL of each dilution as added to duplicate membrane devices and allowed to hydrostatically flow through the device under standard temperature and pressure (~15 minutes). As control, a set of biotinylated die were placed in wells of a 48-well plate and exposed to the same SA-AP dilutions without shaking or other agitation (Sessile incubation) for 15 minutes. After target incubation sensors were rinsed with PBS-ET, removed from the devices, and transferred to wells of the same 48-well plate. All sensors were then incubated with 500 µL of 1-Step NBT/BCIP bottle (Thermo Fisher, 34070), then imaged via standard phase microscopy at 4X magnification to quantitate substrate color development. Membrane images were analyzed for color saturation using ImageJ and Microsoft Excel for further analysis. Figure 9 shows the resulting net color development for duplicate sensors after exposure.

Study B: Protein G Detection via IgG decorated sensors. Immunoglobulin G deposition. 100 nm SiN thick Epichlorohydrin treated die were fixed into a custom 4-port microfluidic assembly for flow experiments. A 5 mg/mL solution of mouse IgG (Rockland D609-0200) was prepared in PBS-ET (1x PBS, .05% Tween 20, 5 mM EDTA) and then 200 µL of the fluid was exposed to the die for 30 minutes. The coating was then blocked with 5% FBS in PBS-ET for 30 minutes, using 200 µL of blocking solution in the device.

Flow experiments. After surface treatment, one die was exposed to each treatment condition, where either Protein G (1 µM, Rockwell PG00-00) or Protein G AP (1 um, EMD Millipore 539305-500UG) were flowed over the membrane (40 µL/min) or through the membrane (20 µL /min over, 20 µL /min through) for 30 minutes. After the flow experiments, the die were rinsed in the device with PBS-ET (80 µL /min for 10 min).

Fluorescence Reaction. To visualize adsorbed protein species, individual die were removed from the microfluidic assembly and all chips were labeled using a 10 mM solution of 4-MUP (Sigma M8168-1G) prepared in pH 7.8 TRIS for 15 minutes (200 µL). Following incubation with reaction substrate, the product was collected and aspirated into a 384-well plate, then read using the well-scan mode of a SpectraMax I3 multimode plate reader (Molecular Devices) at excitation and emission wavelengths for 4-MUP (360/440 nm). After which, the raw mean fluorescence intensity was exported to Microsoft Excel for further analysis. Figure 10 shows the results from this experiment, where a net signal increase of 4.4x was measured for sensors where partial flow-through was applied during target incubation.

### EXAMPLE 4

This example provides a description of exemplary fluidic devices and methods for their use in the present disclosure

Figures 11 and 12 show tangential flow and normal flow, respectively, fluidic devices of the present disclosure incorporating silicon nanomembrane chips.

Figure 11 shows a tangential flow-based fluidic device for incorporating nanomembrane filters. A prototype Fluidic Module with polycarbonate fluidic channels in the body and elastomeric gaskets for filter integration was fabricated by 3D-printing. CAD modeling software was used to render a prototype device (A) suitable for multi-material 3D-printing (B-C). Computational fluid dynamics analysis was performed on the design to verify surface velocities (D), system pressure (E) and sheer stress (F) to ensure such exemplary prototypes would be suitable fluidic devices for the methods of the present disclosure.

Figure 12 shows a representative fluidic device incorporating a nanomembrane filter, wherein the nanomembrane filter is integrated into a centrifuge tube insert fluidic device for dead-end (normal) flow filtration purposes. Figure 12A-F shows representative filter devices incorporating silicon nitride membranes that may employ one or more non-fouling coatings as previously described. Figure H shows a series of representative nanomembranes fabricated using similar fabrication processes. As an example, a three-window membrane comprising three 0.7x3 mm suspended membranes, disposed on a silicon substrate of 5.4x5.4 mm and 0.3 mm thickness. The three 0.7x3 mm silicon nitride membranes further comprise a plurality of 8x50 µm openings patterned and etched through the 400 nm thick silicon nitride membranes. Conventional photolithography, reactive ion etching, and wet chemistry through-wafer etching were used to fabricate such microslit filters.

### EXAMPLE 5

This example provides a description of exemplary fluidic membranes and corresponding physical properties for their use in the present disclosure.

Figures 13-15 show various examples of silicon nanomembranes of the present disclosure as imaged by electron microscopy and further provide summaries of the physical properties of such exemplary silicon nanomembranes.

Figure 13 shows images taken via Electron Microscopy of a range of Silicon Nitride membranes. (A) shows a 400 nm thick microporous SiN membrane of 25.9% porosity decorated with 8.2-micron diameter pores at regular intervals. (B) shows a 400 nm thick microslit membrane of 26.8% porosity with 3.5-micron wide slits. (C) shows a 200 nm thick SiN membrane of 27.2% porosity and 282 nm pores at regular intervals. Finally, (D) shows a 400 nm SiN membrane of 6.2% porosity comprised of 454 nm wide slits.

Figure 14 shows a further image study of micropores as evaluated by electron microscopy. (A) shows a 400 nm thick SiN membrane of 22.1% porosity containing 2.8 micron diameter pores. (B) shows a 400 nm thick SiN membrane of 10.5% porosity containing 682 nm diameter pores. (C) shows a 400 nm thick SiN membrane of 25.5% porosity containing 552 nm diameter pores.

Figure 15 shows a series of nanoporous nitride membranes fabricated using a range of membrane thicknesses, pore diameters, and porosities. (A, B) Show a series of 100 nm thick membranes decorated with either 51 nm pores and 13.9% porosity, or 56.5 nm pores and 16.5% porosity respectively. Images (C-F) show a series of nanomembranes of 50 nm nominal thickness decorated with a range of pore diameters and porosities as follows [C; 83 nm pores, 23.4% porosity. D; 42.8 nm pores, 6% porosity. E; 33.4 nm pores, 6.3% porosity. F; 46.7 nm pores, 31.9% porosity].

The invention is defined by the appended claims.

## Claims

1. A method of preparing, detecting, or assaying an analyte of a sample, comprising:
contacting the sample with a fluidic device comprising a functionalized silicon membrane, wherein the fluidic device isolates one or more analytes of interest from the sample; wherein the functionalized silicon membrane is a functionalized silicon nanomembrane chosen from a nanoporous silicon nitride membrane, a microporous silicon nitride membrane, a microslit silicon nitride membrane, and a microporous silicon oxide membrane;
passing a wash solution through the fluidic device; and
i) eluting the isolated analyte of interest;
transferring the eluted analyte of interest to a storage vessel or analytical instrument; and
performing one or more analytical assays on the eluted analyte of interest; or
ii) passing a solution of one or more detection reagents through the fluidic device; and
measuring a signal of one or more detection reagents; or
iii) extracting nucleic acids from the analyte captured by the fluidic device;
performing a sequencing and/or amplification reaction, wherein reagents for such reactions are passed into the fluidic device;
passing a solution of one or more detection reagents through the device;
measuring a signal of one or more amplification and/or sequencing reaction products; and
the method being **characterized in that** it further comprises a step of functionalizing a silicon membrane to form the functionalized silicon membrane by:
contacting the silicon membrane with a chemical oxidation reagent;
contacting the silicon membrane with an epihalohydrin;
contacting the silicon membrane with a catalyst; and
contacting the silicon membrane with one or more biomolecules.

2. The method of claim 1, wherein the fluidic device further comprises one or more fluidic channels and/or chambers in fluidic contact with one or more membrane surfaces, one or more apertures having one or more surfaces, a plurality of nanopores, micropores, or microslits of the membranes;
wherein, optionally, at least a first and second fluidic channels and/or chambers are in fluidic contact with each other via the one or more apertures and the plurality of nanopores, micropores, or microslits;
wherein, further optionally, the contacting comprises contacting the sample with a first membrane surface and a first fluidic channel or chamber; or
wherein, further optionally, the contacting comprises contacting the sample with a second membrane surface, the one or more apertures, and a second fluidic channel or chamber.

3. The method of claim 1, wherein washing comprises addition of a buffer solution of specified pH, salt, detergent, and/or carrier biomolecule concentration; or
wherein the eluting step comprises chemical denaturation, mechanical denaturation, thermal denaturation, photolysis of a liable bond, reverse flow, or a combination thereof.

4. The method of claim 1, wherein adding detection reagent comprises sequential or concurrent addition of one or more solutions of biomolecule conjugate, a chromogenic substrate, a chemiluminescent substrate, a co-reagent, or a combination thereof; or
wherein adding detection reagent comprises sequential or concurrent addition of at least one or more non-conjugated detection reagents, at least one or more conjugated detection reagents, a chromogenic substrate, a chemiluminescent substrate, a co-reagent, or a combination thereof.

5. The method of claim 1, wherein measuring a signal of one or more detection reagents comprises an optical modality for one or more emission, luminescence, and/or absorbance signal at a defined wavelength or range thereof; or
wherein measuring the signal of one or more detection reagents comprises a plasmonic-enhanced optical modality for one or more emission, luminescence, and/or absorbance signal at a defined wavelength or range thereof; or
wherein the measuring step comprises using electronic interrogation by one or more amperometric or impedimetric methods.

6. The method of claim 1, wherein performing the sequencing and/or amplification reaction comprises the addition of one or more solutions of buffer, salts, detergents, deoxyribonucleotide triphosphates (dNTPs), enzymes, or a combination thereof;
wherein, optionally, thermal cycling is performed in the fluidic device; or
wherein measuring the signal of one or more amplification and/or sequencing reaction products comprises detection of fluorophore incorporating reaction products, release of fluorophores, fluorophore-bound reaction products, chromophore-bound reaction products, or a combination thereof.

7. The method of claim 1, further comprising sequential or concurrent addition of one or more solution of a redox agent, a biomolecule conjugated to a redox agent, or a combination thereof; or
further comprising sequential or concurrent addition of one or more solution of detection reagents, wherein the detection reagents are at least one or more non-conjugated detection reagent, at least one or more conjugated detection reagent, a redox agent, or a combination thereof.

8. The method of claim 1, further comprising contacting the silicon membrane with a spacer compound prior to contacting the silicon membrane with one or more biomolecules, wherein the spacer compound comprises one or amine group, an aliphatic group having two or more carbons, and one or more additional reactive groups.

9. The method of claim 1, further comprising functionalization of the silicon membrane with any optional gas-phase and/or solution-phase non-fouling groups and/or surface property modifying groups.

10. The method of claim 1, further comprising selective functionalization of at least a first membrane surface, at least a second membrane surface, one or more apertures, or one or more intra-pore or intra-slit surfaces, or a combination thereof.

11. The method of claim 1, wherein the functionalized silicon membrane comprises one or more surfaces, one or more opposing surfaces, and a plurality of nanopores, micropores, or microslits passing therebetween;
optionally wherein the nanopores or micropores have a diameter, or the microslits have a width of 11 nm to 10 µm, or optionally
having a nanopore, a micropore, or a microslit density of 10² to 10¹⁰ pores/mm²; or
wherein the functionalized silicon membrane has a thickness of 20 nm to 10 µm.

12. The method of claim 1, wherein contacting the one or more biomolecules further comprises the disposition of the one or more biomolecules in solution onto any membrane surface and/or aperture surface;
wherein, optionally, the one or more biomolecules in solution comprises a solution of the same biomolecule or a solution comprising different biomolecules; or
optionally further comprising disposition of an optional passivation solution and/or stabilizer solution.

13. The method of claim 12, wherein the disposition of the one or more biomolecules in solution comprises using a bulk solution phase process such that the entire or substantially entire membrane surface and/or aperture surface is similarly disposed with the biomolecule in solution; or
wherein the disposition of the one or more biomolecules in solution comprises using a photolithographic, microstamping, or other surface-contact transfer technique, such that the biomolecule solution is disposed in a regular, uniform pattern(s) onto discrete membrane surfaces and/or aperture surfaces.

14. The method of claim 13, wherein the disposition of one or more biomolecules in solution comprises using a discrete liquid dispensing technique, such that droplet volumes of 10 pL to 10 µL are disposed as a circular feature of diameter corresponding to dispensed volume and surface properties of the membrane and/or aperture surfaces; or
comprising continuous disposition of droplets onto any membrane surface and/or aperture, such that a line of length equal to or less than the total width of the membrane and/or aperture is disposed with one or more biomolecules in solution; or
further comprising the continuous disposition of one or more biomolecules in solution as continuous lines on at least a first membrane surface, at least a second membrane surface, and/or one or more aperture surfaces, such that multiple surfaces are successively disposed with any degree of repetition and iteration; or
further comprising the discrete disposition of one or more biomolecule solutions as discrete droplets onto at least a first membrane surface, at least a second membrane surface, and/or aperture surface, such that multiple such surfaces are successively disposed with multiple droplets and any degree of repetition and iteration; or
further comprising unique or similar disposition of one or more biomolecules in solution onto at least a first membrane surface, at least a second membrane surface, and/or one or more aperture surfaces, with any degree of selectivity, repetition and iteration.

15. The method of claim 1, wherein, optionally, the sample comprises a biological, food, environmental, and/or industrial sample, or a combination thereof.

## Patentansprüche

1. Verfahren zum Vorbereiten, Detektieren oder Untersuchen eines Analyten einer Probe, umfassend:
Inkontaktbringen der Probe mit einer fluidischen Vorrichtung, die eine funktionalisierte Siliziummembran umfasst, wobei die fluidische Vorrichtung einen oder mehrere Analyten von Interesse aus der Probe isoliert; wobei die funktionalisierte Siliziummembran eine funktionalisierte Silizium-Nanomembran ist, ausgewählt aus einer nanoporösen Siliziumnitridmembran, einer mikroporösen Siliziumnitridmembran, einer Mikroschlitz-Siliziumnitridmembran und einer mikroporösen Siliziumoxidmembran;
Leiten einer Waschlösung durch die fluidische Vorrichtung; und
i) Eluieren des isolierten Analyten von Interesse;
Überführen des eluierten Analyten von Interesse in ein Lagergefäß oder analytisches Instrument; und
Durchführen eines oder mehrerer analytischer Assays an dem eluierten Analyten von Interesse; oder
ii) Leiten einer Lösung eines oder mehrerer Detektionsreagenzien durch die fluidische Vorrichtung; und
Messen eines Signals eines oder mehrerer Detektionsreagenzien; oder
iii) Extrahieren von Nukleinsäuren aus dem durch die fluidische Vorrichtung eingefangenen Analyten;
Durchführen einer Sequenzier- und/oder Amplifikationsreaktion,
wobei Reagenzien für solche Reaktionen in die fluidische Vorrichtung geleitet werden;
Leiten einer Lösung eines oder mehrerer Detektionsreagenzien durch die Vorrichtung;
Messen eines Signals eines oder mehrerer Amplifikations-und/oder Sequenzierreaktionsprodukte; und
wobei das Verfahren **dadurch gekennzeichnet ist, dass** es ferner einen Schritt des Funktionalisierens einer Siliziummembran umfasst, um die funktionalisierte Siliziummembran zu bilden durch:
Inkontaktbringen der Siliziummembran mit einem chemischen Oxidationsreagenz;
Inkontaktbringen der Siliziummembran mit einem Epihalohydrin;
Inkontaktbringen der Siliziummembran mit einem Katalysator; und
Inkontaktbringen der Siliziummembran mit einem oder mehreren Biomolekülen.

2. Verfahren nach Anspruch 1, wobei die fluidische Vorrichtung ferner einen oder mehrere fluidische Kanäle und/oder Kammern in fluidischem Kontakt mit einer oder mehreren Membranoberflächen, eine oder mehrere Öffnungen mit einer oder mehreren Oberflächen, eine Vielzahl von Nanoporen, Mikroporen oder Mikroschlitzen der Membranen umfasst;
wobei optional wenigstens ein erster und zweiter fluidischer Kanal und/oder Kammer über die eine oder die mehreren Öffnungen und die Vielzahl von Nanoporen, Mikroporen oder Mikroschlitzen miteinander in fluidischem Kontakt stehen;
wobei ferner optional das Inkontaktbringen das Inkontaktbringen der Probe mit einer ersten Membranoberfläche und einem ersten fluidischen Kanal oder einer ersten fluidischen Kammer umfasst; oder
wobei ferner optional das Inkontaktbringen das Inkontaktbringen der Probe mit einer zweiten Membranoberfläche, der einen oder den mehreren Öffnungen und einem zweiten fluidischen Kanal oder einer zweiten fluidischen Kammer umfasst.

3. Verfahren nach Anspruch 1, wobei das Waschen die Zugabe einer Pufferlösung mit spezifiziertem pH-Wert, spezifizierter Salz-, Detergens- und/oder Trägerbiomolekülkonzentration umfasst; oder wobei der Eluierungsschritt chemische Denaturierung, mechanische Denaturierung, thermische Denaturierung, Photolyse einer labilen Bindung, Rückfluss oder eine Kombination davon umfasst.

4. Verfahren nach Anspruch 1, wobei das Hinzufügen von Detektionsreagenz die sequentielle oder gleichzeitige Zugabe einer oder mehrerer Lösungen von Biomolekülkonjugat, einem chromogenen Substrat, einem chemilumineszierenden Substrat, einem Co-Reagenz oder einer Kombination davon umfasst; oder
wobei das Hinzufügen von Detektionsreagenz die sequentielle oder gleichzeitige Zugabe von wenigstens einem oder mehreren nicht konjugierten Detektionsreagenzien, wenigstens einem oder mehreren konjugierten Detektionsreagenzien, einem chromogenen Substrat, einem chemilumineszierenden Substrat, einem Co-Reagenz oder einer Kombination davon umfasst.

5. Verfahren nach Anspruch 1, wobei das Messen eines Signals eines oder mehrerer Detektionsreagenzien eine optische Modalität für ein oder mehrere Emissions-, Lumineszenz- und/oder Absorptionssignale bei einer definierten Wellenlänge oder einem Bereich davon umfasst; oder
wobei das Messen des Signals eines oder mehrerer Detektionsreagenzien eine plasmonisch verstärkte optische Modalität für ein oder mehrere Emissions-, Lumineszenz- und/oder Absorptionssignale bei einer definierten Wellenlänge oder einem Bereich davon umfasst; oder
wobei der Messschritt die Verwendung elektronischer Abfrage durch ein oder mehrere amperometrische oder impedimetrische Verfahren umfasst.

6. Verfahren nach Anspruch 1, wobei das Durchführen der Sequenzier- und/oder Amplifikationsreaktion die Zugabe einer oder mehrerer Lösungen von Puffer, Salzen, Detergenzien, Desoxyribonukleotidtriphosphaten (dNTPs), Enzymen oder einer Kombination davon umfasst;
wobei optional thermisches Zyklisieren in der fluidischen Vorrichtung durchgeführt wird; oder
wobei das Messen des Signals eines oder mehrerer Amplifikations-und/oder Sequenzierreaktionsprodukte die Detektion von Fluorophor einbauenden Reaktionsprodukten, Freisetzung von Fluorophoren, fluorphorgebundenen Reaktionsprodukten, chromophorgebundenen Reaktionsprodukten oder einer Kombination davon umfasst.

7. Verfahren nach Anspruch 1, ferner umfassend sequentielle oder gleichzeitige Zugabe einer oder mehrerer Lösungen eines Redoxmittels, eines an ein Redoxmittel konjugierten Biomoleküls oder einer Kombination davon; oder
ferner umfassend sequentielle oder gleichzeitige Zugabe einer oder mehrerer Lösungen von Detektionsreagenzien, wobei die Detektionsreagenzien wenigstens ein oder mehrere nicht konjugierte Detektionsreagenzien, wenigstens ein oder mehrere konjugierte Detektionsreagenzien, ein Redoxmittel oder eine Kombination davon sind.

8. Verfahren nach Anspruch 1, ferner umfassend Inkontaktbringen der Siliziummembran mit einer Spacerverbindung vor dem Inkontaktbringen der Siliziummembran mit einem oder mehreren Biomolekülen, wobei die Spacerverbindung eine oder mehrere Amingruppen, eine aliphatische Gruppe mit zwei oder mehr Kohlenstoffen und eine oder mehrere zusätzliche reaktive Gruppen umfasst.

9. Verfahren nach Anspruch 1, ferner umfassend Funktionalisierung der Siliziummembran mit beliebigen optionalen gasphasen- und/oder lösungsphasen-Anti-Fouling-Gruppen und/oder oberflächeneigenschaftsmodifizierenden Gruppen.

10. Verfahren nach Anspruch 1, ferner umfassend selektive Funktionalisierung wenigstens einer ersten Membranoberfläche, wenigstens einer zweiten Membranoberfläche, einer oder mehrerer Öffnungen oder einer oder mehrerer Intra-Poren- oder Intra-Schlitz-Oberflächen oder einer Kombination davon.

11. Verfahren nach Anspruch 1, wobei die funktionalisierte Siliziummembran eine oder mehrere Oberflächen, eine oder mehrere gegenüberliegende Oberflächen und eine Vielzahl von dazwischen verlaufenden Nanoporen, Mikroporen oder Mikroschlitzen umfasst;
optional wobei die Nanoporen oder Mikroporen einen Durchmesser aufweisen oder die Mikroschlitze eine Breite von 11 nm bis 10 µm aufweisen, oder optional
mit einer Nanoporen-, Mikroporen- oder Mikroschlitzdichte von 10² bis 10¹⁰ Poren/mm²; oder
wobei die funktionalisierte Siliziummembran eine Dicke von 20 nm bis 10 µm aufweist.

12. Verfahren nach Anspruch 1, wobei das Inkontaktbringen des einen oder der mehreren Biomoleküle ferner das Aufbringen des einen oder der mehreren Biomoleküle in Lösung auf eine beliebige Membranoberfläche und/oder Öffnungsoberfläche umfasst;
wobei optional das eine oder die mehreren Biomoleküle in Lösung eine Lösung desselben Biomoleküls oder eine Lösung, die unterschiedliche Biomoleküle umfasst, umfassen; oder
optional ferner umfassend Aufbringen einer optionalen Passivierungslösung und/oder Stabilisatorlösung.

13. Verfahren nach Anspruch 12, wobei das Aufbringen des einen oder der mehreren Biomoleküle in Lösung das Verwenden eines Bulk-Lösungsphasenprozesses umfasst, sodass die gesamte oder im Wesentlichen die gesamte Membranoberfläche und/oder Öffnungsoberfläche ähnlich mit dem Biomolekül in Lösung belegt wird; oder
wobei das Aufbringen des einen oder der mehreren Biomoleküle in Lösung das Verwenden eines photolithographischen, Mikrostempel-oder anderen Oberflächenkontakttransferverfahrens umfasst, sodass die Biomoleküllösung in einem regelmäßigen, einheitlichen Muster(n) auf diskrete Membranoberflächen und/oder Öffnungsoberflächen aufgebracht wird.

14. Verfahren nach Anspruch 13, wobei das Aufbringen eines oder mehrerer Biomoleküle in Lösung das Verwenden einer diskreten Flüssigkeitsabgabetechnik umfasst, sodass Tröpfchenvolumina von 10 pL bis 10 µL als ein kreisförmiges Merkmal mit einem Durchmesser aufgebracht werden, der dem abgegebenen Volumen und den Oberflächeneigenschaften der Membran- und/oder Öffnungsoberflächen entspricht; oder
umfassend kontinuierliches Aufbringen von Tröpfchen auf eine beliebige Membranoberfläche und/oder Öffnung, sodass eine Linie mit einer Länge gleich oder kleiner als die Gesamtbreite der Membran und/oder Öffnung mit einem oder mehreren Biomolekülen in Lösung belegt wird; oder
ferner umfassend das kontinuierliche Aufbringen eines oder mehrerer Biomoleküle in Lösung als kontinuierliche Linien auf wenigstens eine erste Membranoberfläche, wenigstens eine zweite Membranoberfläche und/oder eine oder mehrere Öffnungsoberflächen, sodass mehrere Oberflächen nacheinander mit einem beliebigen Grad an Wiederholung und Iteration belegt werden; oder
ferner umfassend das diskrete Aufbringen einer oder mehrerer Biomoleküllösungen als diskrete Tröpfchen auf wenigstens eine erste Membranoberfläche, wenigstens eine zweite Membranoberfläche und/oder Öffnungsoberfläche, sodass mehrere solche Oberflächen nacheinander mit mehreren Tröpfchen und einem beliebigen Grad an Wiederholung und Iteration belegt werden; oder
ferner umfassend einzigartiges oder ähnliches Aufbringen eines oder mehrerer Biomoleküle in Lösung auf wenigstens eine erste Membranoberfläche, wenigstens eine zweite Membranoberfläche und/oder eine oder mehrere Öffnungsoberflächen mit einem beliebigen Grad an Selektivität, Wiederholung und Iteration.

15. Verfahren nach Anspruch 1, wobei optional die Probe eine biologische, Nahrungsmittel-, Umwelt- und/oder Industrieprobe oder eine Kombination davon umfasst.

## Revendications

1. Procédé de préparation, de détection ou de dosage d'un analyte d'un échantillon, comprenant :
la mise en contact de l'échantillon avec un dispositif fluidique comprenant une membrane de silicium fonctionnalisée, dans lequel le dispositif fluidique isole un ou plusieurs analytes d'intérêt de l'échantillon ; dans lequel la membrane de silicium fonctionnalisée est une nanomembrane de silicium fonctionnalisée choisie parmi une membrane de nitrure de silicium nanoporeuse, une membrane de nitrure de silicium microporeuse, une membrane de nitrure de silicium à microfentes et une membrane d'oxyde de silicium microporeuse ;
l'introduction d'une solution de lavage à travers le dispositif fluidique ; et
i) l'élution de l'analyte d'intérêt isolé ;
le transfert de l'analyte élué d'intérêt dans un récipient de stockage ou un instrument analytique ; et
la réalisation d'un ou de plusieurs dosages analytiques sur l'analyte élué d'intérêt ; ou
ii) l'introduction d'une solution d'un ou de plusieurs réactifs de détection à travers le dispositif fluidique ; et
la mesure d'un signal d'un ou de plusieurs réactifs de détection ; ou
iii) l'extraction d'acides nucléiques de l'analyte capturé par le dispositif fluidique ;
la réalisation d'une réaction de séquençage et/ou d'amplification, dans lequel des réactifs de ces réactions sont introduits dans le dispositif fluidique ;
l'introduction d'une solution d'un ou de plusieurs réactifs de détection à travers le dispositif ;
la mesure d'un signal d'un ou de plusieurs produits de réaction d'amplification et/ou de séquençage ; et
le procédé étant **caractérisé en ce qu'**il comprend également une étape de fonctionnalisation d'une membrane de silicium pour former la membrane de silicium fonctionnalisée par :
la mise en contact de la membrane de silicium avec un réactif d'oxydation chimique ;
la mise en contact de la membrane de silicium avec une épihalohydrine ;
la mise en contact de la membrane de silicium avec un catalyseur ; et
la mise en contact de la membrane de silicium avec une ou plusieurs biomolécules.

2. Procédé selon la revendication 1, dans lequel le dispositif fluidique comprend également un ou plusieurs canaux et/ou chambres fluidiques en contact fluidique avec une ou plusieurs surfaces membranaires, une ou plusieurs ouvertures ayant une ou plusieurs surfaces, une pluralité de nanopores, de micropores ou de microfentes des membranes ;
dans lequel, éventuellement, au moins un premier et second canaux fluidiques et/ou une première et seconde chambres fluidiques sont en contact fluidique l'un avec l'autre via les une ou plusieurs ouvertures et la pluralité de nanopores, micropores ou microfentes ;
dans lequel, également éventuellement, la mise en contact comprend la mise en contact de l'échantillon avec une première surface membranaire et un premier canal fluidique ou une première chambre fluidique ; ou
dans lequel, également éventuellement, la mise en contact comprend la mise en contact de l'échantillon avec une seconde surface membranaire, les une ou plusieurs ouvertures et un second canal fluidique ou une seconde chambre fluidique.

3. Procédé selon la revendication 1, dans lequel le lavage comprend l'ajout d'une solution tampon de pH, de sel, de détergent et/ou de concentration de biomolécule porteuse spécifiés ; ou
dans lequel l'étape d'élution comprend une dénaturation chimique, une dénaturation mécanique, une dénaturation thermique, une photolyse d'une liaison fragile, un flux inverse ou une combinaison de celles-ci.

4. Procédé selon la revendication 1, dans lequel l'ajout du réactif de détection comprend l'ajout séquentiel ou simultané d'une ou de plusieurs solutions de conjugué de biomolécule, d'un substrat chromogène, d'un substrat chimioluminescent, d'un co-réactif ou d'une combinaison de ceux-ci ; ou
dans lequel l'ajout du réactif de détection comprend l'ajout séquentiel ou simultané d'au moins un ou plusieurs réactifs de détection non conjugués, d'au moins un ou plusieurs réactifs de détection conjugués, d'un substrat chromogène, d'un substrat chimioluminescent, d'un co-réactif ou d'une combinaison de ceux-ci.

5. Procédé selon la revendication 1, dans lequel la mesure d'un signal d'un ou de plusieurs réactifs de détection comprend une modalité optique pour un ou plusieurs signaux d'émission, de luminescence et/ou d'absorbance à une longueur d'onde définie ou à une plage de longueurs d'onde définie ; ou
dans lequel la mesure du signal d'un ou de plusieurs réactifs de détection comprend une modalité optique à amplification plasmonique pour un ou plusieurs signaux d'émission, de luminescence et/ou d'absorption à une longueur d'onde définie ou à une plage de longueurs d'onde définie ; ou
dans lequel l'étape de mesure comprend l'utilisation d'une interrogation électronique par un ou plusieurs procédés ampérométriques ou impédimétriques.

6. Procédé selon la revendication 1, dans lequel la réalisation de la réaction de séquençage et/ou d'amplification comprend l'ajout d'une ou de plusieurs solutions de tampon, de sels, de détergents, de désoxyribonucléotides triphosphates (dNTP), d'enzymes ou d'une combinaison de ceux-ci ;
dans lequel, éventuellement, un cycle thermique est réalisé dans le dispositif fluidique ; ou
dans lequel la mesure du signal d'un ou de plusieurs produits de réaction d'amplification et/ou de séquençage comprend la détection de produits de réaction incorporant un fluorophore, la libération de fluorophores, de produits de réaction liés à un fluorophore, de produits de réaction liés à un chromophore ou une combinaison de ceux-ci.

7. Procédé selon la revendication 1, comprenant également l'ajout séquentiel ou simultané d'une ou de plusieurs solutions d'un agent redox, d'une biomolécule conjuguée à un agent redox, ou d'une combinaison de ceux-ci ; ou
comprenant également l'ajout séquentiel ou simultané d'une ou de plusieurs solutions de réactifs de détection, dans lequel les réactifs de détection sont au moins un ou plusieurs réactifs de détection non conjugués, au moins un ou plusieurs réactifs de détection conjugués, un agent redox ou une combinaison de ceux-ci.

8. Procédé selon la revendication 1, comprenant également la mise en contact de la membrane de silicium avec un composé espaceur avant la mise en contact de la membrane de silicium avec une ou plusieurs biomolécules, dans lequel le composé espaceur comprend un ou plusieurs groupes amine, un groupe aliphatique ayant deux carbones ou plus, et un ou plusieurs groupes réactifs supplémentaires.

9. Procédé selon la revendication 1, comprenant également la fonctionnalisation de la membrane de silicium avec tous groupes anti-encrassement optionnels en phase gazeuse et/ou en phase solution et/ou groupes modifiant les propriétés de surface.

10. Procédé selon la revendication 1, comprenant également la fonctionnalisation sélective d'au moins une première surface membranaire, d'au moins une seconde surface membranaire, d'une ou de plusieurs ouvertures, ou d'une ou de plusieurs surfaces intra-pores ou intra-fentes, ou une combinaison de celles-ci.

11. Procédé selon la revendication 1, dans lequel la membrane de silicium fonctionnalisée comprend une ou plusieurs surfaces, une ou plusieurs surfaces opposées et une pluralité de nanopores, de micropores ou de microfentes passant entre elles ;
éventuellement, dans lequel les nanopores ou les micropores ont un diamètre, ou les microfentes ont une largeur de 11 nm à 10 µm, ou éventuellement
présentant une densité de nanopores, de micropores ou de microfentes de 10² à 10¹⁰ pores/mm² ; ou
dans lequel la membrane de silicium fonctionnalisée a une épaisseur de 20 nm à 10 µm.

12. Procédé selon la revendication 1, dans lequel la mise en contact des une ou plusieurs biomolécules comprend également la disposition des une ou plusieurs biomolécules en solution sur une surface membranaire et/ou une surface d'ouverture ;
dans lequel, éventuellement, les une ou plusieurs biomolécules en solution comprennent une solution de la même biomolécule ou une solution comprenant des biomolécules différentes ; ou
comprenant éventuellement également la disposition d'une solution de passivation et/ou d'une solution stabilisatrice facultative.

13. Procédé selon la revendication 12, dans lequel la disposition des une ou plusieurs biomolécules en solution comprend l'utilisation d'un procédé en phase solution en vrac tel que la totalité ou la quasi-totalité de la surface membranaire et/ou de la surface d'ouverture soit disposée de la même manière avec la biomolécule en solution ; ou
dans lequel la disposition des une ou plusieurs biomolécules en solution comprend l'utilisation d'une technique de transfert par photolithographie, micro-estampage ou autre technique de contact de surface, de sorte que la solution de biomolécules soit disposée selon un ou plusieurs motifs réguliers et uniformes sur des surfaces membranaires et/ou des surfaces d'ouverture discrètes.

14. Procédé selon la revendication 13, dans lequel la disposition d'une ou de plusieurs biomolécules en solution comprend l'utilisation d'une technique de distribution de liquide discrète, de sorte que des volumes de gouttelettes de 10 pL à 10 µL soient disposés sous forme d'un élément circulaire de diamètre correspondant au volume distribué et aux propriétés de surface des surfaces membranaires et/ou des surfaces d'ouverture ; ou
comprenant la disposition continue de gouttelettes sur une surface membranaire et/ou une surface d'ouverture, de sorte qu'une ligne de longueur égale ou inférieure à la largeur totale de la membrane et/ou de l'ouverture soit disposée avec une ou plusieurs biomolécules en solution ; ou
comprenant également la disposition continue d'une ou de plusieurs biomolécules en solution sous forme de lignes continues sur au moins une première surface membranaire, au moins une seconde surface membranaire et/ou une ou plusieurs surfaces d'ouverture, de sorte que plusieurs surfaces soient disposées successivement avec un degré quelconque de répétition et d'itération ; ou
comprenant également la disposition discrète d'une ou de plusieurs solutions de biomolécules sous forme de gouttelettes discrètes sur au moins une première surface membranaire, au moins une seconde surface membranaire et/ou une surface d'ouverture, de sorte que plusieurs de ces surfaces soient successivement recouvertes de plusieurs gouttelettes, avec un degré quelconque de répétition et d'itération ; ou
comprenant également la disposition unique ou similaire d'une ou de plusieurs biomolécules en solution sur au moins une première surface membranaire, au moins une seconde surface membranaire et/ou une ou plusieurs surfaces d'ouverture, avec un degré quelconque de sélectivité, de répétition et d'itération.

15. Procédé selon la revendication 1, dans lequel, éventuellement, l'échantillon comprend un échantillon biologique, alimentaire, environnemental et/ou industriel, ou une combinaison de ceux-ci.
